# FASCICULE DE BREVET EUROPEEN

(11) **EP 1 885 730 B1**
(45) Date de publication et mention de la délivrance du brevet: **22.04.2009**
(21) Numéro de dépôt: 05798554.1
(22) Date de dépôt: 24.08.2005
(51) Int. Cl.: C07F 7/08, A61K 8/18, C08G 77/04

(54) **DERIVES SILICIES DE SULFONE MEROCYANINE ; COMPOSITIONS PHOTOPROTECTRICES LES CONTENANT ; UTILISATION COMME FILTRE UV**
SILANMEROCYANINSULPHONDERIVATE, PHOTOPROTEKTIVE ZUSAMMENSETZUNGEN DAMIT, VERWENDUNG DAVON ALS UV-FILTER
SILANE MEROCYANINE SULPHONE DERIVATIVES; PHOTOPROTECTING COMPOSITIONS CONTAINING SAME; USE THEREOF AS UV FILTER

(30) Priorité: 20.09.2004 FR 0452093; 29.09.2004 US 613967 P
(43) Date de publication de la demande: 13.02.2008
(73) Titulaire: L'ORÉAL, 75008 Paris (FR)
(72) Inventeur: RICHARD, Hervé, F-93420 Villepinte (FR)
(74) Mandataire: Miszputen, Laurent
(86) Numéro de dépôt international: PCT/FR2005/002129
(87) Numéro de publication internationale: WO 2006/032741

(56) Documents cités:
- EP-A- 1 371 654
- WO-A-20/04006878
- US-A1- 2002 081 271
- US-A1- 2004 059 119

## Description

La présente invention concerne de nouveaux composés silaniques, siloxaniques ou polysiloxaniques de sulfone mérocyanine ainsi que leurs utilisations en cosmétique comme filtres UVA longs.

La présente invention concerne également des compositions cosmétiques à usage topique, en particulier destinées à la photoprotection de la peau et/ou des cheveux, comprenant une quantité efficace d'au moins un dérivé ou un mélange de dérivés silaniques, siloxaniques ou polysiloxaniques de sulfone mérocyanine.

On sait que les radiations de longueurs d'onde comprises entre 280 nm et 400 nm permettent le brunissement de l'épiderme humain, et que les radiations de longueurs d'onde comprises entre 280 et 320 nm connues sous la dénomination de radiations UV-B, provoquent des érythèmes et des brûlures cutanées qui peuvent nuire au développement du bronzage naturel.

On sait également que les rayons UV-A, de longueurs d'onde comprises entre 320 et 400 nm, provoquant le brunissement de la peau, sont susceptibles d'induire une altération de celle-ci, notamment dans le cas d'une peau sensible et/ou continuellement exposée au rayonnement solaire. Les rayons UV-A entraînent en particulier une perte d'élasticité de la peau et l'apparition de rides conduisant à un vieillissement cutané prématuré. Ils favorisent le déclenchement de la réaction érythémateuse ou amplifient cette réaction chez certains sujets et peuvent même être à l'origine de réactions phototoxiques ou photoallergiques. Ainsi, pour des raisons esthétiques et cosmétiques telles que la conservation de l'élasticité naturelle de la peau, de plus en plus de personnes désirent contrôler l'effet des rayons UV-A sur leur peau. On entend par facteur de protection solaire le rapport du temps d'irradiation nécessaire pour atteindre le seuil érythématogène en présence du filtre testé au temps d'irradiation nécessaire pour atteindre ce même seuil en l'absence de filtre.

De nombreux composés organiques destinés à la protection contre le rayonnement UV-A et/ou UV-B de la peau ont été proposés à ce jour.

La plupart d'entre eux sont des composés aromatiques absorbant le rayonnement UV dans la zone comprise entre 280 nm et 315 nm, ou dans la zone comprise entre 315 nm et 400 nm et au-delà, ou bien dans l'ensemble de ces deux zones. Ils sont le plus souvent formulés dans des compositions antisolaires qui se présentent sous forme d'émulsions huile-dans-eau ou eau-dans-huile Les filtres organiques, généralement lipophiles ou hydrophiles, sont présents sous forme dissoute, dans l'une ou l'autre de ces phases, en des quantités appropriées pour obtenir le facteur de protection solaire (FPS) recherché.

Outre leur pouvoir filtrant du rayonnement solaire, les composés photoprotecteurs recherchés doivent également présenter de bonnes propriétés cosmétiques, une bonne solubilité dans les solvants usuels et en particulier dans les corps gras tels que les huiles et les graisses, ainsi qu'une bonne résistance à l'eau et à la transpiration (rémanence) et une très bonne photostabilité.

Parmi tous les composés qui ont été préconisés à cet effet, on peut citer notamment la famille de filtres particulièrement intéressante constituée par des dérivés de mérocyanines carbonés décrite dans le brevet US4195999 ou la demande WO 2004/006878. Ces composés présentent de très bonnes propriétés filtrantes dans l'UV-A long, mais possèdent une solubilité peu satisfaisante dans les solvants organiques usuels et notamment les corps gras tels que les huiles et une photostabilité sur certaines familles de mérocyanines non satisfaisante.

On connaît également des dérivés silaniques ou siloxaniques de phenylsulfone acrylonitrile dans la demande EP1371654 ainsi que leurs utilisations comme filtres UVA. Les demandes de brevet US20040059119 et US2002/0081271 décrvent également des dérivés siliconés à fonction benzotriazole dans des formulations solaires

La demanderesse a découvert de manière surprenante une nouvelle famille de dérivés silaniques, siloxaniques ou polysiloxaniques de sulfone mérocyanine présentant de bonnes propriétés filtrantes dans l'UV-A Long, une très bonne solubilité dans les solvants organiques usuels ainsi que d'excellentes propriétés cosmétiques par rapport aux mérocyanines de la demande WO 2004/006878.

L'invention concerne une nouvelle famille de dérivés silaniques, siloxaniques ou polysiloxaniques de sulfone mérocyanine que l'on définira plus loin en détail.

L'invention concerne également une composition cosmétique ou dermatologique, destinée à la photoprotection des matières kératiniques, contenant dans un milieu physiologiquement acceptable au moins un composé silanique, siloxanique ou polysiloxanique de sulfone mérocyanine que l'on définira plus loin en détail.

On entend par «milieu physiologiquement acceptable», un milieu non toxique et susceptible d'être appliqué sur la peau, les lèvres, les cheveux, les cils, les sourcils les ongles. La composition de l'invention peut constituer notamment une composition cosmétique ou dermatologique.

D'autres objets apparaîtront à la lumière de la description.

Les composés conformes à la présente invention sont choisis parmi ceux qui répondent à l'une ou à l'autre des formules (1), (2) ou (3) suivantes : 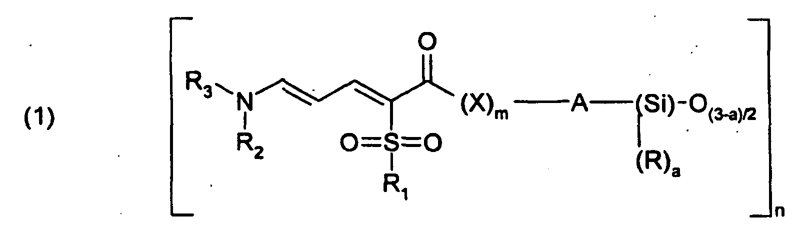 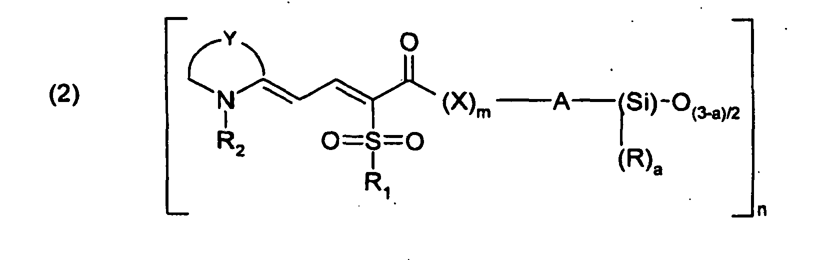 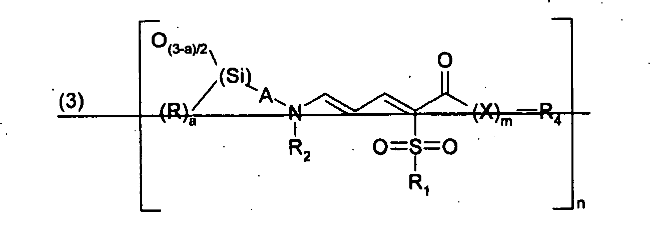 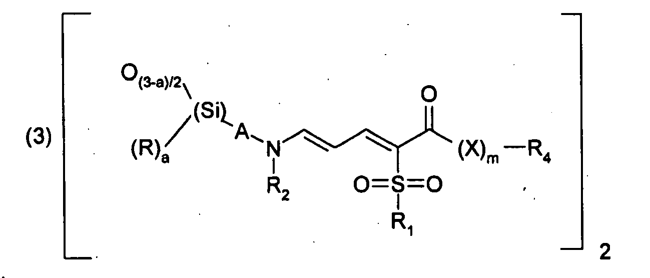 dans laquelle :
m = 0 ou 1,
X = représente -O-, -NR₅-, -SO₂NH-, avec R₅ qui représente l'hydrogène ou un radical alkyle en C₁-C₅,
Y est un radical divalent alkyle en C₁-C₅ éventuellement substitué par des radicaux alkyles en C₁-C₄ et/ou contenant des atomes -O-, -S-, ou par un groupe -NR₁,
R, identiques ou différents représentent un radical alkyle en C₁-C₂₀, linéaire ou ramifié et éventuellement halogéné, un radical aryle en C₆-C₁₂ ou un groupe alkoxy en C₁-C₁₀, a = 0 à 3,
R₁ représente un radical alkyle en C₁-C₂₀, linéaire ou ramifié et éventuellement halogéné ou un radical aryle en C₆-C₁₂,
R₂, R₃ = identiques ou différents représentent H, un radical alkyle en C₁-C₂₄, cycloalkyle en C₃-C₁₀, aryle en C₆-C₂₀ substitué par des groupements alkyle ou alcoxy, et R₂, R₃ ensembles avec l'atome d'azote peuvent former un cycle en C₄-C₆ éventuellement interrompu par des atomes d'oxygène ou -NH-, avec le fait que R₂, R₃ ne peuvent ensemble représenter un atome d'hydrogène,
R₄ est un groupe alkyle divalent
A est un radical divalent choisi parmi méthylène, éthylène ou un groupe répondant à l'une des formules (4), (5) ou (6) suivantes :

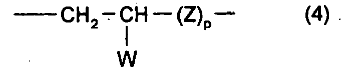

   -CH=CH-(Z)ₚ- (5) 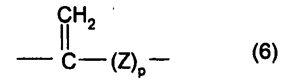
dans lesquelles Z est un radical alkylène en C₁-C₆ linéaire ou ramifié, saturé ou insaturé, éventuellement substitué par un radical hydroxyle ou un radical alkyle en C₁-C₈, linéaire ou ramifié, saturé ou insaturé,
W représente un atome d'hydrogène; un radical hydroxyle ou un radical alkyle en C₁-C₈, linéaire ou ramifié, saturé ou insaturé,
p est 0 ou 1,
n = 1 à 4 et dans le cas ou n = 2, R₁, R₂ ou R₃ est un groupe alkyle divalent ou R₂ et R₃ ensembles avec l'atome d'azote peuvent former un cycle en C₄-C₆ éventuellement interrompu par des atomes d'oxygène ou -NH-.

Les composés des formules (1), (2) et (3) peuvent être présents sous les formes isomériques E,E-, E,Z- ou Z,Z-.

En plus des unités de formule -A-(Si)(R)ₐ(O)_{(3-a)/2}, l'organosiloxane peut comporter des unités de formule (R)_{b}-(Si)(O)_{(4-b)/2} dans lesquelles :
R a la même signification que dans les formules (1) à (3),
b = 1, 2 ou 3,

De manière préférée, les groupements -(Si)(R)ₐ(O)_{(3-a)/2} peuvent être représentés par les formules (7), (8) ou (9) suivantes : 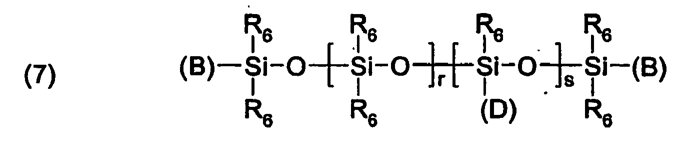 ou

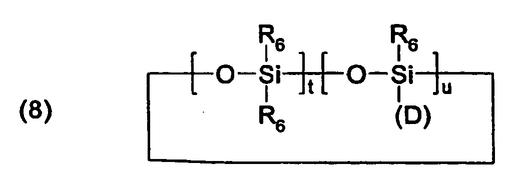

(9) (D)-Si(R₆)₃

dans lesquelles :
(D) est liant entre la chaîne siliconée au groupement A des chromophores des formules (1) à (3),
R₆, identiques ou différents, sont choisis parmi les radicaux alkyles linéaires ou ramifiés en C₁-C₃₀, phényle, 3,3,3-trifluoropropyle et triméthylsilyloxy, au moins 80% en nombre des radicaux R₆ étant méthyle,
(B), identiques ou différents sont choisis parmi les radicaux R₆ et le radical A
r est un nombre entier compris entre 0 et 200 inclusivement, et s est un nombre entier compris entre 0 et 50 inclusivement, et si s = 0, au moins l'un des deux symboles (B) désigne A,
u est un nombre entier compris entre 1 et 10 inclusivement, et t est un nombre entier compris entre 0 et 10 inclusivement, étant entendu que t + u est égal ou supérieur à 3.

Dans les formules (1) à (3) ci-dessus, les radicaux alkyles peuvent être linéaires ou ramifiés, saturés ou insaturés et choisis notamment au sein des radicaux méthyle, éthyle, n-propyle, isopropyle, n-butyle, isobutyle, ter.-butyle, n-amyle, isoamyle, néopentyle, n-hexyle, n-heptyle, n-octyle, éthyl-2 hexyle et tert-octyle. Le radical alkyle particulièrement préféré est le radical méthyle.

Dans les formules (1) à (3) ci-dessus, les radicaux aryles sont de préférence phényle ou tolyle.

Y est plus particulièrement un groupement d'atomes dont le résultat réside en la formation d'un cycle oxazolidine, d'un cycle pyrrolidine, d'un cycle thiazolidine ou d'un bicycle indoline.

Les diorganosiloxanes linéaires ou cycliques de formule (7) ou (8) rentrant dans le cadre de la présente invention, sont des oligomères ou polymères statistiques présentant de préférence au moins l'une, et encore plus préférentiellement l'ensemble, des caractéristiques suivantes :
- R₆ est préférentiellement méthyle,
- B est préférentiellement méthyle (cas des composés linéaires de formule (7)),

A titre d'exemples de composés de formule (1) particulièrement préférés, on citera les produits de formules (a) à (e) suivantes :

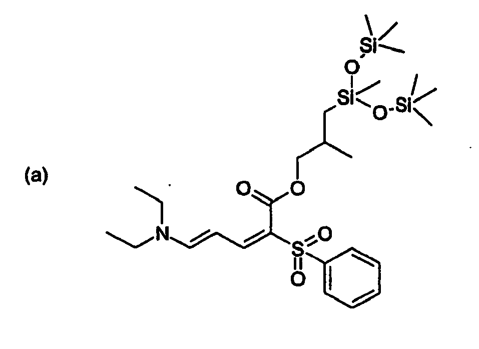

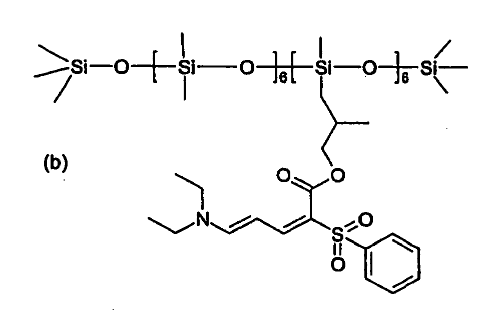

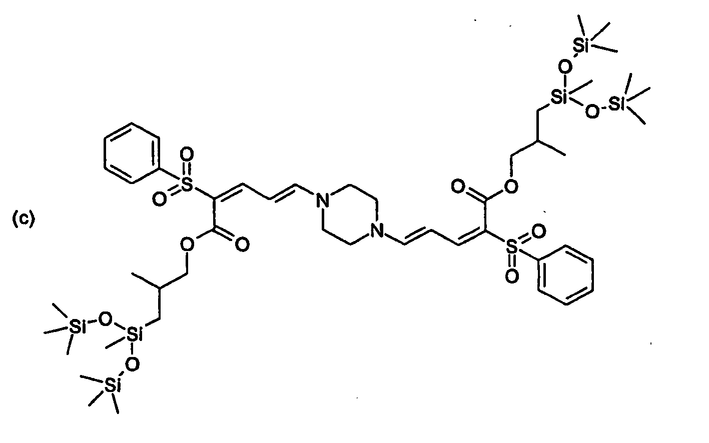

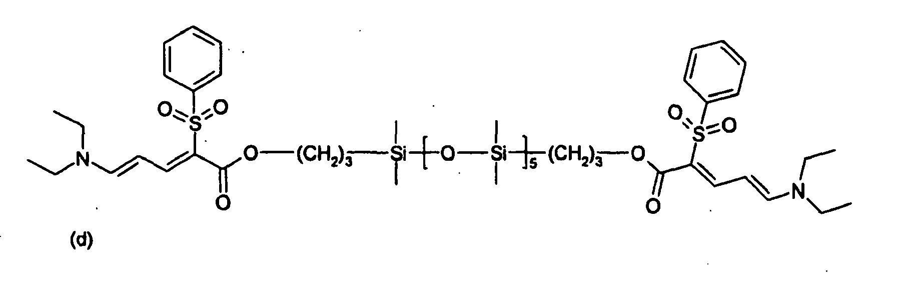

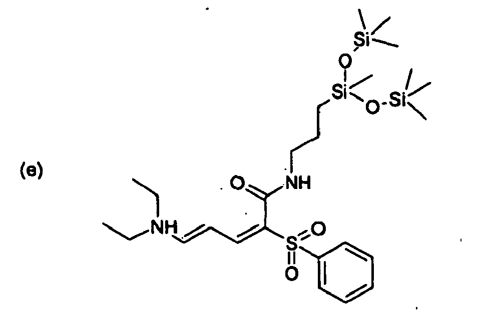

A titre d'exemples de composés de formule (2) particulièrement préférés, on citera le produit de formule (f) suivante :

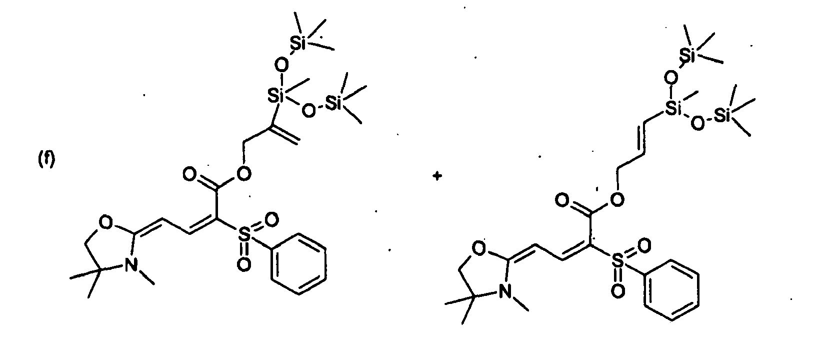

Les composés conformes à la présente invention sont également choisis parmi les produits de formules (g) à (k) suivantes :

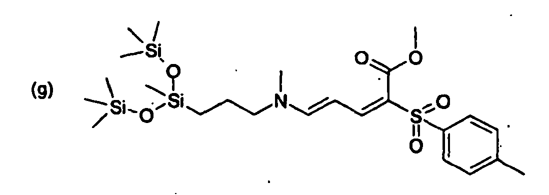

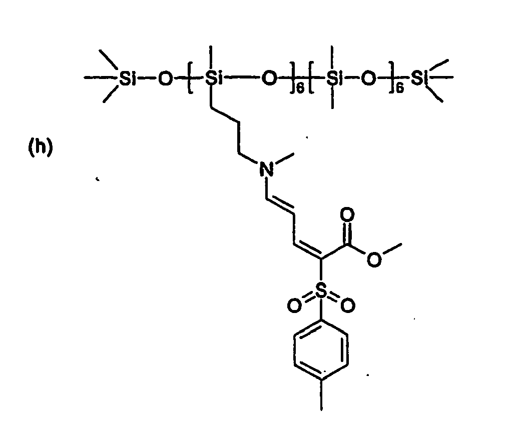

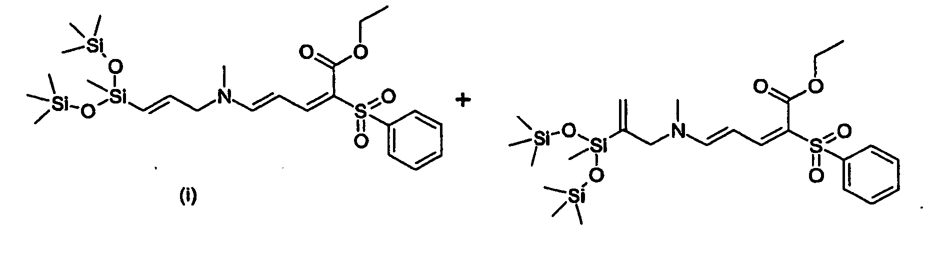

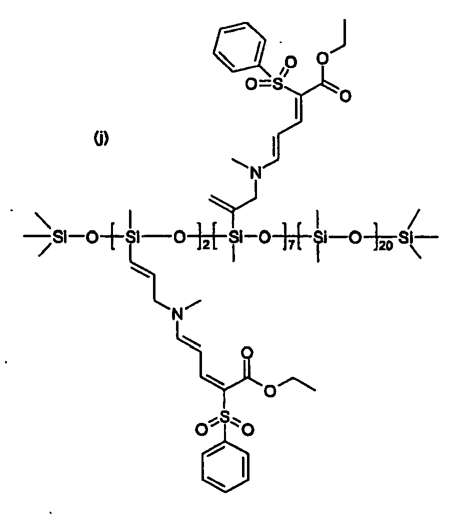

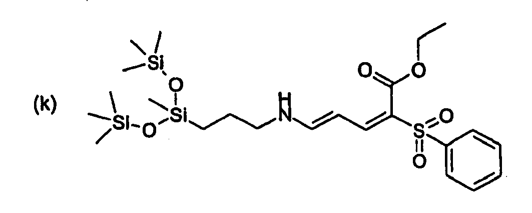

Les dérivés de formules (1) peuvent être préparés selon une méthode décrite dans les brevets US 4045229 et US 4195999 selon le schéma suivant : 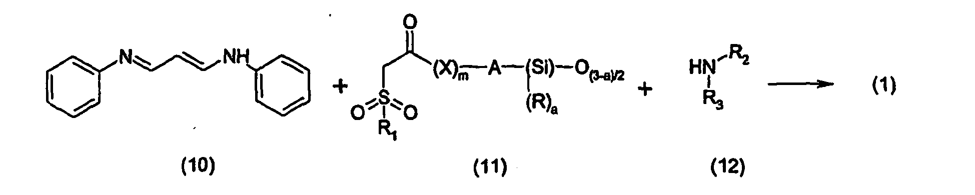 dans lequel les radicaux R, R₁, R₂, R₃, A, X, a et m ont la signification des formules ci-dessus.

Les dérivés de formules (2) peuvent être préparés selon une méthode décrite dans le brevet WO 0020388 selon le schéma suivant : 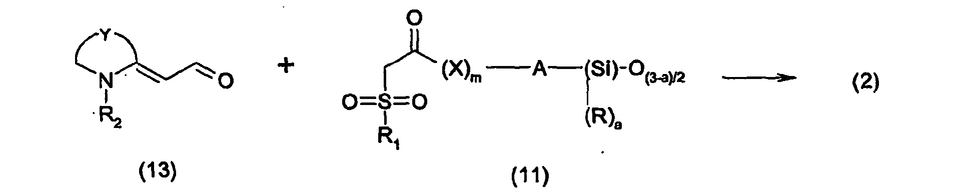 dans lequel les radicaux R, R₁, R₂, A, X, Y, a et m ont la signification des formules ci-dessus.

Les dérivés de formules (3) peuvent être préparés selon une méthode décrite dans les brevets US 4045229 et US 4195999 selon le schéma suivant : 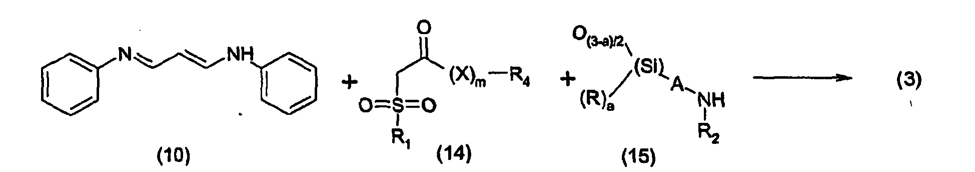 dans lequel les radicaux R, R₁, R₂, R₄, A, X, a et m ont la signification des formules ci-dessus.

Les composés de formule (11) peuvent être obtenus classiquement en mettant en oeuvre une réaction d'hydrosilylation à partir d'un dérivé siloxanique ou silanique de formules (7) à (8) dans lesquelles par exemple tous les (C) sont des atomes d'hydrogène, ce dérivé est dénommé par la suite dérivé à SiH et un dérivé insaturé selon le schéma réactionnel suivant : 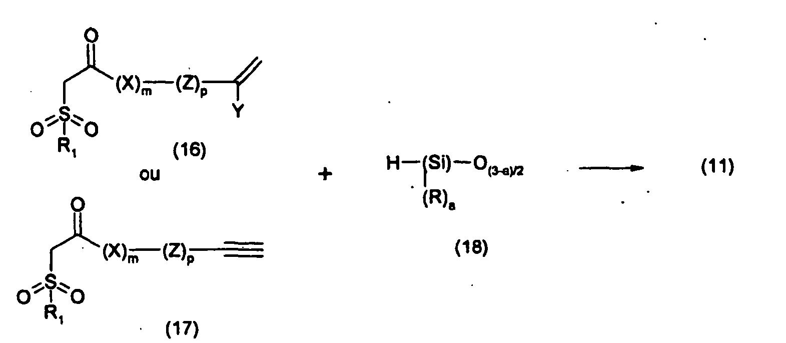 dans lequel les radicaux R, R₁, A, X, Y, Z, a, m et p ont la signification des formules ci-dessus.

Les groupes SiH peuvent être présents dans la chaîne et/ou aux extrémités de la chaîne. Ces dérivés à SiH sont des produits bien connus dans l'industrie des silicones et sont généralement disponibles dans le commerce. Ils sont par exemple décrits dans les brevets américains US 3220972, US 3697473 et US 4340709.

De la même façon, les dérivés de formule (15) peuvent être obtenus selon le schéma suivant : 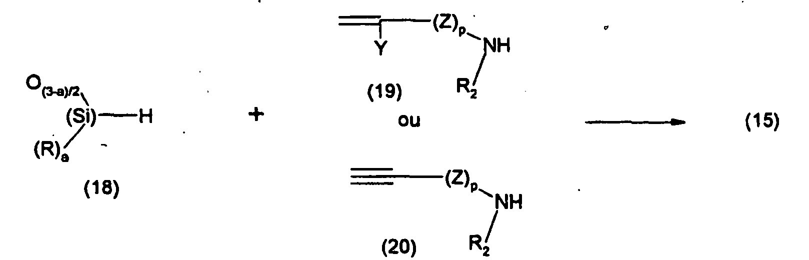 dans lequel les radicaux R, R₂, Y, Z, a et p ont la signification des formules ci-dessus.

Le ou les composés silaniques, siloxaniques ou polysiloxaniques de sulfone mérocyanine conformes à l'invention peuvent être présents dans les compositions de l'invention dans des proportions allant de 0,01 % à 20 % en poids, de préférence de 0,1 % à 10 % en poids, par rapport au poids total de la composition.

Les compositions conformès à l'invention peuvent comporter en plus d'autres filtres UV organiques ou inorganiques complémentaires actifs dans l'UVA et/ou l'UVB hydrosolubles ou liposolubles ou bien insolubles dans les solvants cosmétiques couramment utilisés.

Les filtres organiques complémentaires sont notamment choisis parmi les anthranilates ; les dérivés cinnamiques ; les dérivés salicyliques, les dérivés du camphre ; les dérivés de triazine autres que ceux de l'invention tels que ceux décrits dans les demandes de brevet US 4367390, EP863145, EP517104, EP570838, EP796851, EP775698, EP878469, EP933376, EP507691, EP507692, EP790243, EP944624 ; les dérivés de la benzophénone ; les dérivés de β,β-diphénylacrylate ; les dérivés de benzotriazole; les dérivés de benzalmalonate ; les dérivés de benzimidazole ; les imidazolines ; les dérivés bis-benzoazolyle tels que décrits dans les brevets EP669323 et US 2,463,264; les dérivés de l'acide, p-aminobenzoïque (PABA) ; les dérivés de méthylène bis-(hydroxyphényl benzotriazole) tels que décrits dans les demandes US 5,237,071, US 5,166,355, GB2303549, DE 197 26 184 et EP893119 ; les polymères filtres et silicones filtres tels que ceux décrits notamment dans la demande WO-93104665 ; les dimères dérivés d'α-alkylstyrène tels que ceux décrits dans la demande de brevet DE19855649 ; les 4,4-diarylbutadiènes tels que décrits dans les demandes EP0967200, DE19746654, DE19755649, EP-A-1008586, EP1133980 et EP133981 et leurs mélanges.

Comme exemples de filtres organiques complémentaires, on peut citer ceux désignés ci-dessous sous leur nom INCI :

### Dérivés de l'acide para-aminobenzoigue:

PABA,
Ethyl PABA,
Ethyl Dihydroxypropyl PABA,
Ethylhexyl Diméthyl PABA vendu notamment sous le nom « ESCALOL 507 » par ISP,
Glyceryl PABA,
PEG-25 PABA vendu sous le nom « UVINUL P25 » par BASF,

### Dérivés salicyligues :

Homosalate vendu sous le nom « Eusolex HMS » par Rona/EM Industries,
Ethylhexyl Salicylate vendu sous le nom « NEO HELIOPAN OS » par HAARMANN et REIMER,
Dipropyleneglycol Salicylate vendu sous le nom « DIPSAL » par SCHER,
TEA Salicylate, vendu sous le nom « NEO HELIOPAN TS » par HAARMANN et REIMER,

### Dérivés cinnamiques :

Ethylhexyl Methoxycinnamate vendu notamment sous le nom commercial « PARSOL MCX » par HOFFMANN LA ROCHE,
Isopropyl Methoxy cinnamate,
Isoamyl Methoxy cinnamate vendu sous le nom commercial « NEO HELIOPAN E 1000 » par HAARMANN et REIMER,
DEA Methoxycinnamate,
Diisopropyl Methylcinnamate,
Glyceryl Ethylhexanoate Dimethoxycinnamate

### Dérivés de β,β'-diphénylacrylate :

Octocrylene vendu notamment sous le nom commercial « UVINUL N539 » par BASF,
Etocrylene, vendu notamment sous le nom commercial « UVINUL N35 » par BASF,

### Dérivés de la benzophénone :

Benzophenone-1 vendu sous le nom commercial « UVINUL 400 » par BASF,
Benzophenone-2 vendu sous le nom commercial « UVINUL D50 » par BASF
Benzophenone-3 ou Oxybenzone, vendu sous le nom commercial « UVINUL M40 » par BASF,
Benzophenone-4 vendu sous le nom commercial « UVINUL MS40 » par BASF,
Benzophenone-5
Benzophenone-6 vendu sous le nom commercial « Helisorb 11 » par Norquay
Benzophenone-8 vendu sous le nom commercial « Spectra-Sorb UV-24 » par American Cyanamid
Benzophenone-9 vendu sous le nom commercial« UVINUL DS-49» par BASF, Benzophenone-12
Diethylamino Hydroxybenzoyl Hexyl Benzoate vendu sous le nom commercial « UVINUL A PLUS» par BASF,

### Dérivés du benzylidène camphre :

3-Benzylidene camphor fabriqué sous le nom « MEXORYL SD» par CHIMEX,
4-Methylbenzylidene camphor vendu sous le nom « EUSOLEX 6300 » par MERCK ,
Benzylidene Camphor Sulfonic Acid fabriqué sous le nom « MEXORYL SL» par CHIMEX,
Camphor Benzalkonium Methosulfate fabriqué sous le nom « MEXORYL SO » par CHIMEX,
Terephthalylidene Dicamphor Sulfonic Acid fabriqué sous le nom « MEXORYL SX » par CHIMEX,
Polyacrylamidomethyl Benzylidene Camphor fabriqué sous le nom « MEXORYL SW » par CHIMEX,

### Dérivés du phenyl benzimidazole :

Phenylbenzimidazole Sulfonic Acid vendu notamment sous le nom commercial « EUSOLEX 232 » par MERCK,
Disodium Phenyl Dibenzimidazole Tetra-sulfonate vendu sous le nom commercial commercial « NEO HELIOPAN AP » par HAARMANN et REIMER,

### Dérivés de la triazine :

Bis-Ethylhexyloxyphenol Methoxyphenyl Triazine vendu sous le nom commercial «TINOSORB S » par CIBA GEIGY,
Ethylhexyl triazone vendu notamment sous le nom commercial «UVINUL T150 » par BASF,
Diethylhexyl Butamido Triazone vendu sous le nom commercial « UVASORB HEB » par SIGMA 3V,

### Dérivés du phenyl benzotriazole :

Drometrizole Trisiloxane vendu sous le nom « Silatrizole » par RHODIA CHIMIE Methylène bis-Benzotriazolyl Tetramethylbutylphénol, vendu sous forme solide sous le nom commercial « MIXXIM BB/100 » par FAIRMOUNT CHEMICAL ou sous forme micronisé en dispersion aqueuse sous le nom commercial « TINOSORB M » par CIBA SPECIALTY CHEMICALS,

### Dérivés anthraniliques :

Menthyl anthranilate vendu sous le nom commercial commercial « NEO HELIOPAN MA » par HAARMANN et REIMER,

### Dérivés d'imidazolines :

Ethylhexyl Dimethoxybenzylidene Dioxoimidazoline Propionate,

### Dérivés du benzalmalonate :

Polyorganosiloxanes à fonction benzalmalonate tels que le Polysilicone-15 vendu sous la dénomination commerciale « PARSOL SLX » par HOFFMANN LA ROCHE

### Dérivés de 4,4-diarylbutadiène :

- 1,1-dicarboxy (2,2'-diméthyl-propyl)-4,4-diphénylbutadiène
et leurs mélanges.

### Dérivés de benzoxazole :

2,4-bis-[5-1(diméthylpropyl)benzoxazol-2-yl-(4-phenyl)-imino]-6-(2-ethylhexyl)-imino-1,3,5-triazine vendu sous le nom d'Uvasorb K2A par Sigma 3V.
et leurs mélanges.

Les filtres UV organiques complémentaires préférentiels sont choisis parmi
Ethylhexyl Salicylate,
Homosalate,
Ethylhexyl Methoxycinnamate
Octocrylene,
Phenylbenzimidazole Sulfonic Acid,
Disodium Phenyl Dibenzimidazole Tetra-sulfonate,
Benzophenone-3,
Benzophenone-4,
Benzophenone-5,
2-(4-diethylamino-2-hydroxybenzoyl)-benzoate de n-hexyle.
4-Methylbenzylidene camphor,
Terephthalylidene Dicamphor Sulfonic Acid,
Anisotriazine,
Ethylhexyl triazone,
Diethylhexyl Butamido Triazone,
Methylène bis-Benzotriazolyl Tetramethylbutylphénol
Drometrizole Trisiloxane
Polysilicone-15
1,1-dicarboxy (2,2'-diméthyl-propyl)-4,4-diphénylbutadiène
2,4-bis-[5-1(diméthylpropyl)benzoxazol-2-yl-(4-phenyl)-imino]-6-(2-ethylhexyl)-imino-1,3,5-triazine et leurs mélanges.

Les agents photoprotecteurs complémentaires inorganiques sont choisis parmi des pigments et plus préférentiellement encore des nanopigments (taille moyenne des particules primaires : généralement entre 5 nm et 100 nm, de préférence entre 10 nm et 50 nm) d'oxydes métalliques traités ou non comme par exemple des nanopigments d'oxyde de titane (amorphe ou cristallisé sous forme rutile et/ou anatase), de fer, de zinc, de zirconium ou de cérium.

Les nanopigments traités sont des pigments qui ont subi un ou plusieurs traitements de surface de nature chimique, électronique, mécanochimique et/ou mécanique avec des composés tels que décrits par exemple dans Cosmetics & Toiletries, Février 1990, Vol. 105, p 53-64, tels que des aminoacides, de la cire d'abeille, des acides gras, des alcools gras, des tensio-actifs anioniques, des lécithines, des sels de sodium, potassium, zinc, fer, ou aluminium d'acides gras, des alcoxydes métalliques (de titane ou d'aluminium), du polyéthylène, des silicones, des protéines (collagène, élastine) des alcanolamines, des oxydes de silicium, des oxydes métalliques, de l'hexamétaphosphate de sodium, de l'alumine ou de la glycérine.

Les nanopigments traités peuvent être plus particulièrement des oxydes de titane traités par:
- la silice et l'alumine tels que les produits « Microtitanium Dioxide MT 500 SA » et « Microtitanium Dioxide MT 100 SA » de la société TAYCA, et les produits « Tioveil Fin », « Tioveil OP », « Tioveil MOTG » et « Tioveil IPM » de la société TIOXIDE,
- l'alumine et le stéarate d'aluminium tels que le produit « Microtitanium Dioxide MT 100 T » de la société TAYCA,
- l'alumine et le laurate d'aluminium tels que le produit « Microtitanium Dioxide MT 100 S » de la société TAYCA,
- des oxydes de fer et le stéarate de fer tels que le produit « Microtitanium Dioxide MT 100 F » de la société TAYCA,
- la silice, l'alumine et la silicone tels que les produits « Microtitanium Dioxide MT 100 SAS », « Microtitanium Dioxide MT 600 SAS » et « Microtitanium Dioxide MT 500 SAS » de la société TAYCA,
- l'hexamétaphosphate de sodium tels que le produit « Microtitanium Dioxide MT 150 W » de la société TAYCA,
- l'octyltriméthoxysilane tels que le produit « T-805 » de la société DEGUSSA,
- l'alumine et l'acide stéarique tels que le produit « UVT-M160 » de la société KEMIRA,
- l'alumine et la glycérine tels que le produit « UVT-M212» de la société KEMIRA,
- l'alumine et la silicone tels que le produit « UVT-M262» de la société KEMIRA.

D'autres nanopigments d'oxyde de titane traités avec une silicone sont de préférence le TiO₂ traité par l'octyl triméthyl silane et dont la taille moyenne des particules élémentaires est comprise entre 25 et 40 nm tel que celui vendu sous la dénomination commerciale "T 805" par la société DEGUSSA SILICES, le TiO2 traité par un polydiméthylsiloxane et dont la taille moyenne des particules élémentaires est de 21 nm tel que celui vendu sous la dénomination commerciale "70250 CARDRE UF TiO2SI3" par la société CARDRE, le TiO2 anatase/rutile traité par un polydiméthylhydrogénosiloxane et dont la taille moyenne des particules élémentaires est de 25 nm tel que celui vendu sous la dénomination commerciale "MICRO TITANIUM DIOXYDE USP GRADE HYDROPHOBIC" par la société COLOR TECHNIQUES.

Les nanopigments d'oxyde de titane non enrobés sont par exemple vendus par la société TAYCA sous les dénominations commerciales "MICROTITANIUM DIOXIDE MT 500 B" ou "MICROTITANIUM DIOXIDE MT600 B", par la société DEGUSSA sous la dénomination "P 25", par la société WACKHER sous la dénomination "Oxyde de titane transparent PW", par la société MIYOSHI KASEI sous la dénomination "UFTR", par la société TOMEN sous la dénomination "ITS" et par la société TIOXIDE sous la dénomination "TIOVEIL AQ".

Les nanopigments d'oxyde de zinc non enrobés, sont par exemple
- ceux commercialisés sous la dénomination "Z-COTE" par la société SUNSMART ;
- ceux commercialisés sous la dénomination "NANOX" par la société ELEMENTIS ;
- ceux commercialisés sous la dénomination "NANOGARD WCD 2025" par la société NANOPHASE TECHNOLOGIES ;

Les nanopigments d'oxyde de zinc enrobés sont par exemple
- ceux commercialisés sous la dénomination "OXIDE ZINC CS-5" par la société Toshibi (ZnO enrobé par polymethylhydrogenesiloxane) ;
- ceux commercialisés sous la dénomination "NANOGARD ZINC OXIDE FN" par la société NANOPHASE TECHNOLOGIES (en dispersion à 40% dans le Finsolv TN, benzoate d'alcools en C₁₂-C₁₅) ;
- ceux commercialisés sous la dénomination "DAITOPERSION ZN-30" et "DAITOPERSION ZN-50" par la société Daito (dispersions dans cyclopolyméthylsiloxane /polydiméthylsiloxane oxyéthyléné, contenant 30% ou 50% de nano-oxydes de zinc enrobés par la silice et le polyméthylhydrogènesiloxane) ;
- ceux commercialisés sous la dénomination "NFD ULTRAFINE ZNO" par la société Daikin (ZnO enrobé par phosphate de perfluoroalkyle et copolymère à base de perfluoroalkyléthyle en dispersion dans du cyclopentasiloxane) ;
- ceux commercialisés sous la dénomination "SPD-Z1" par la société Shin-Etsu (ZnO enrobé par polymère acrylique greffé silicone, dispersé dans cyclodiméthylsiloxane) ;
- ceux commercialisés sous la dénomination "ESCALOL Z100" par la société ISP (ZnO traité alumine et dispersé dans le mélange methoxycinnamate d'ethylhexyle / copolymère PVP-hexadecene / methicone) ;
- ceux commercialisés sous la dénomination "FUJI ZNO-SMS-10" par la société Fuji Pigment (ZnO enrobé silice et potymethylsilsesquioxane) ;
- ceux commercialisés sous la dénomination "NANOX GEL TN" par la société Elementis (ZnO dispersé à 55% dans du benzoate d'alcools en C₁₂-C₁₅ avec polycondensat d'acide hydroxystéarique).

Les nanopigments d'oxyde de cérium non enrobé est vendu sous la dénomination "COLLOIDAL CERIUM OXIDE" par la société RHONE POULENC.

Les nanopigments d'oxyde de fer non enrobés sont par exemple vendus par la société ARNAUD sous les dénominations "NANOGARD WCD 2002 (FE 45B)", "NANOGARD IRON FE 45 BL AQ", "NANOGARD FE 45R AQ, "NANOGARD WCD 2006 (FE 45R)", ou par la société MITSUBISHI sous la dénomination "TY-220".

Les nanopigments d'oxyde de fer enrobés sont par exemple vendus par la société ARNAUD sous les dénominations "NANOGARD WCD 2008 (FE 45B FN)", "NANOGARD WCD 2009 (FE 45B 556)", "NANOGARD FE 45 BL 345", "NANOGARD FE 45 BL", ou par la société BASF sous la dénomination "OXYDE DE FER TRANSPARENT".

On peut également citer les mélanges d'oxydes métalliques, notamment de dioxyde de titane et de dioxyde de cérium, dont le mélange équipondéral de dioxyde de titane et de dioxyde de cérium enrobés de silice, vendu par la société IKEDA sous la dénomination "SUNVEIL A", ainsi que le mélange de dioxyde de titane et de dioxyde de zinc enrobé d'alumine, de silice et de silicone tel que le produit "M 261" vendu par la société KEMIRA ou enrobé d'alumine, de silice et de glycérine tel que le produit "M 211" vendu par la société KEMIRA.

Les nanopigments peuvent être introduits dans les compositions selon l'invention tels quels ou sous forme de pâte pigmentaire, c'est-à-dire en mélange avec un dispersant, comme décrit par exemple dans le document GB-A-2206339.

Les agents photoprotecteurs additionnels sont généralement présents dans les compositions selon l'invention dans des proportions allant de 0,01 à 20% en poids par rapport au poids total de la composition, et de préférence allant de 0,1 à 10% en poids par rapport au poids total de la composition.

Les compositions selon l'invention peuvent également contenir des agents de bronzage et/ou de brunissage artificiels de la peau (agents autobronzants), et plus particulièrement la dihydroxyacétone (DHA). Ils sont présents de préférence dans des quantité allant 0,1 à 10% en poids par rapport au poids total de la composition.

Les compositions conformes à la présente invention peuvent comprendre en outre des adjuvants cosmétiques classiques notamment choisis parmi les corps gras, les solvants organiques, les épaississants ioniques ou non ioniques, hydrophiles ou lipophiles, les adoucissants, les humectants, les opacifiants, les stabilisants, les émollients, les silicones, les agents anti-mousse, les parfums, les conservateurs, les tensioactifs anioniques, cationiques, non-ioniques, zwitterioniques ou amphotères, des actifs, les charges, les polymères, les propulseurs, les agents alcalinisants ou acidifiants ou tout autre ingrédient habituellement utilisé dans le domaine cosmétique et/ou dermatologique.

Les corps gras peuvent être constitués par une huile ou une cire ou leurs mélanges. Par huile, on entend un composé liquide à température ambiante. Par cire, on entend un composé solide ou substantiellement solide à température ambiante, et dont le point de fusion est généralement supérieur à 35°C.

Comme huiles, on peut citer les huiles minérales (paraffine); végétales (huile d'amande douce, de macadamia, de pépin de cassis, de jojoba) ; synthétiques comme le perhydrosqualène, les alcools, les acides ou les esters gras (comme le benzoate d'alcools en C₁₂-C₁₅ vendu sous la dénomination commerciale « Finsolv TN » par la société WITCO, le palmitate d'octyle, le lanolate d'isopropyle, les triglycérides dont ceux des acides caprique/caprylique), les esters et éthers gras oxyéthylénés ou oxypropylénés; les huiles siliconées (cyclométhicone, polydiméthysiloxanes ou PDMS) ou fluorées, les polyalkylènes.

Comme composés cireux, on peut citer la paraffine, la cire de carnauba, la cire d'abeille, l'huile de ricin hydrogénée.

Parmi les solvants organiques, on peut citer les alcools et polyols inférieurs. Ces derniers peuvent être choisis parmi les glycols et les éthers de glycol comme l'éthylène glycol, le propylène glycol, le butylène glycol, le dipropylène glycol ou le diéthylène glycol.

Comme épaississants hydrophiles, on peut citer les polymères carboxyvinyliques tels que les carbopols (carbomers) et les Pemulen (Copolymère acrylate/C10-C30-alkylacrylate) ; les polyacrylamides comme par exemple les copolymères réticulés vendus sous les noms Sepigel 305 (nom C.T.F.A. : polyacrylamide/C13-14 isoparaffin/Laureth 7) ou Simulgel 600 (nom C.T.F.A. : acrylamide / sodium acryloyldimethyltaurate copolymer / isohexadecane / polysorbate 80) par la société Seppic ; les polymères et copolymères d'acide 2-acrylamido 2-méthylpropane sulfonique, éventuellement réticulés et/ou neutralisés, comme le poly(acide 2-acrylamido 2-méthylpropane sulfonique) commercialisé par la société Hoechst sous la dénomination commerciale « Hostacerin AMPS » (nom CTFA : ammonium polyacryldimethyltauramide); les dérivés cellulosiques tels que l'hydroxyéthylcellulose ; les polysaccharides et notamment les gommes telles que la gomme de xanthane ; et leurs mélanges.

Comme épaississants lipophiles, on peut citer les argiles modifiées telles que le l'hectorite et ses dérivés, comme les produits commercialisés sous les noms de Bentone.

Parmi les actifs, on peut citer :
- les agents anti-pollution et/ou agent anti-radicalaire ;
- les agents dépigmentants et/ou des agents pro-pigmentants ;
- les agents anti-glycation ;
- les inhibiteurs de NO-synthase ;
- les agents stimulant la synthèse de macromolécules dermiques ou épidermiques et/ou empêchant leur dégradation ;
- les agents stimulant la prolifération des fibroblastes ;
- les agents stimulant la prolifération des kératinocytes ;
- les agents myorelaxants ;
- les agents tenseurs.
- les agents desquamants ;
- les agents hydratants ;
- les agents anti-inflammatoires ;
- les agents agissant sur le métabolisme énergétique des cellules.
- les agents répulsifs contre les insectes
- les antagonistes de substances P ou de CRGP.

Bien entendu, l'homme de l'art veillera à choisir le ou les éventuels composés complémentaires cités ci-dessus et/ou leurs quantités de manière telle que les propriétés avantageuses attachées intrinsèquement aux compositions conformes à l'invention ne soient pas, ou substantiellement pas, altérées par la ou les adjonctions envisagées.

Les compositions selon l'invention peuvent être préparées selon les techniques bien connues de l'homme de l'art, en particulier celles destinées à la préparation d'émulsions de type huile-dans-eau ou eau-dans-huile. Elles peuvent se présenter en particulier sous forme d'émulsion, simple ou complexe (H/E, E/H, H/E/H ou E/H/E) telle qu'une crème, un lait, ou sous la forme d'un gel ou d'un gel crème, sous la forme d'une lotion, de poudre, de bâtonnet solide et éventuellement être conditionnée en aérosol et se présenter sous forme de mousse ou de spray.

De préférence, les compositions selon l'invention se présentent sous la forme d'une émulsion huile-dans-eau ou eau-dans huile.

Les émulsions contiennent généralement au moins un émulsionnant choisi parmi les émulsionnants amphotères, anioniques, cationiques ou non ioniques, utilisés seuls ou en mélange. Les émulsionnants sont choisis de manière appropriée suivant l'émulsion à obtenir (E/H ou H/E).

Comme tensioactifs émulsionnants utilisables pour la préparation des émulsions E/H, on peut citer par exemple les alkyl esters ou éthers de sorbitane, de glycérol ou de sucres ; les tensioactifs siliconés comme les dimethicone copolyols tels que le mélange de cyclomethicone et de dimethicone copolyol, vendu sous la dénomination « DC 5225 C » par la société Dow Coming, et les alkyl-dimethicone copolyols tels que le Laurylmethicone copolyol vendu sous la dénomination "Dow Coming 5200 Formulation Aid" par la société Dow Coming ; le Cetyl dimethicone copolyol tel que le produit vendu sous la dénomination Abil EM 90R par la société Goldschmidt et le mélange de cétyl diméthicone copolyol, d'isostearate de polyglycérole (4 moles) et de laurate d'hexyle vendu sous la dénomination ABIL WE 09 par la société Goldschmidt. On peut y ajouter aussi un ou plusieurs co-émulsionnants, qui, de manière avantageuse, peuvent être choisis dans le groupe comprenant les esters alkylés de polyol. Comme esters alkylés de polyol, on peut citer notamment les esters de glycérol et/ou de sorbitan et par exemple l'isostéarate de polyglycérol, tel que le produit commercialisé sous la dénomination Isolan GI 34 par la société Goldschmidt, l'isostéarate de sorbitan, tel que le produit commercialisé sous la dénomination Arlacel 987 par la société ICI, l'isostéarate de sorbitan et le glycérol, tel que le produit commercialisé sous la dénomination Arlacel 986 par la société ICI, et leurs mélanges.

Pour les émulsions H/E, on peut citer par exemple comme émulsionnants, les émulsionnants non ioniques tels que les esters d'acides gras et de glycérol oxyalkylénés (plus particulièrement polyoxyéthylénés) ; les esters d'acides gras et de sorbitan oxyalkylénés ; les esters d'acides gras oxyalkylénés (oxyéthylénés et/ou oxypropylénés) ; les éthers d'alcools gras oxyalkylénés (oxyéthylénés et/ou oxypropylénés) ; les esters de sucres comme le stéarate de sucrose ; les éthers d'alcool gras et de sucre, notamment les alkylpolyglucosides (APG) tels que le décylglucoside et le laurylglucoside commercialisés par exemple par la société Henkel sous les dénominations respectives Plantaren 2000 et Plantaren 1200, le cétostéarylglucoside éventuellement en mélange avec l'alcool cétostéarylique, commercialisé par exemple sous la dénomination Montanov 68 par la société Seppic, sous la dénomination Tegocare CG90 par la société Goldschmidt et sous la dénomination Emulgade KE3302 par la société Henkel, ainsi que l'arachidyl glucoside, par exemple sous la forme du mélange d'alcools arachidique et béhénique et d'arachidylglucoside commercialisé sous la dénomination Montanov 202 par la société Seppic. Selon un mode particulier de réalisation de l'invention, le mélange de l'alkylpolyglucoside tel que défini ci-dessus avec l'alcool gras correspondant peut être sous forme d'une composition autoémulsionnante, comme décrit par exemple dans le document WO-A-92/06778.

Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. 13, 238 (1965), FR 2 315 991 et FR 2 416 008).

Les compositions selon l'invention trouvent leur application dans un grand nombre de traitements, notamment cosmétiques, de la peau, des lèvres et des cheveux, y compris le cuir chevelu, notamment pour la protection et/ou le soin de la peau, des lèvres et/ou des cheveux, et/ou pour le maquillage de la peau et/ou des lèvres.

Un autre objet de la présente invention est constitué par l'utilisation des compositions selon l'invention telles que ci-dessus définies pour la fabrication de produits pour le traitement cosmétique de la peau, des lèvres, des ongles, des cheveux, des cils, sourcils et/ou du cuir chevelu, notamment des produits de soin et des produits de maquillage.

Les compositions cosmétiques selon l'invention peuvent par exemple être utilisées comme produit de soin et/ou de protection solaire pour le visage et/ou le corps de consistance liquide à semi-liquide, telles que des laits, des crèmes plus ou moins onctueuses, gel-crèmes, des pâtes. Elles peuvent éventuellement être conditionnées en aérosol et se présenter sous forme de mousse ou de spray.

Les compositions selon l'invention sous forme de lotions fluides vaporisables conformes à l'invention sont appliquées sur la peau ou les cheveux sous forme de fines particules au moyen de dispositifs de pressurisation. Les dispositifs conformes à l'invention sont bien connus de l'homme de l'art et comprennent les pompes non-aérosols ou "atomiseurs", les récipients aérosols comprenant un propulseur ainsi que les pompes aérosols utilisant l'air comprimé comme propulseur. Ces derniers sont décrits dans les brevets US 4,077,441 et US 4,850,517 (faisant partie intégrante du contenu de la description).

Les compositions conditionnées en aérosol conformes à l'invention contiennent en général des agents propulseurs conventionnels tels que par exemple les composés hydrofluorés le dichlorodifluorométhane, le difluoroéthane, le diméthyléther, l'isobutane, le n-butane, le propane, le trichlorofluorométhane. Ils sont présents de préférence dans des quantités allant de 15 à 50% en poids par rapport au poids total de la composition.

Un autre objet de l'invention est l'utilisation d'un composé de formule (1), (2) ou (3) tel que définie ci-dessus dans une composition cosmétique ou dermatologique comme agent filtrant les radiations UV.

Un autre objet de l'invention est l'utilisation d'un composé des formule (1), (2) ou (3) tel que définie ci-dessus dans une composition cosmétique comme agent de contrôle de la variation de la couleur de la peau due aux rayonnements UV.

Un autre objet de l'invention est l'utilisation d'un composé des formule (1), (2) ou (3) tel que définie précédemment comme agent photostabilisant de polymères synthétiques tels que les matières plastiques ou de verres en particulier des verres de lunettes ou des lentilles de contact.

Des exemples concrets, mais nullement limitatifs, illustrant l'invention, vont maintenant être donnés.

### EXEMPLE 1 :

### Préparation du composé (g)

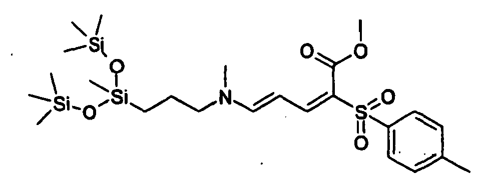

### Première étape : Préparation du méthyl-5-[allyl(méthyl)amino]-2-[(4-méthylphényl)sulfonyl]penta-2,4-diènoate :

On chauffe à 85-90°C dans 5 ml d'anhydride acétique du 3-anilinoacroléine aniline (1,2 g, 5,4x10⁻³ mole) et du para-toluène sulphonylacetate de méthyle (1,48 g, 6,48x10⁻³ mole) pendant 2 heures 30 minutes. On évapore à sec sous pression réduite l'anhydride acétique. L'huile obtenue est reprise dans 5 ml d'éthanol. On y ajoute de la N-méthyl allylamine (1,115 ml, 0,0117 mole) et on porte le mélange au reflux pendant 4 heures 30 minutes. On évapore à sec sous pression réduite l'éthanol. L'huile brun orangé obtenue est purifiée par une colonne chromatographique de silice (éluant : AcOEt / Heptane 20 :80 puis gradient jusque 30:70. On récupère 1,48 g de fractions propres (Rendement : 77%) de méthyl-5-[allyl(méthyl)amino]-2-[(4-méthylphényl)sulfonyl]penta-2,4-diènoate sous forme d'une huile jaune pâle:

| | | |
|---|---|---|
| UV (CH₂Cl₂): | λₘₐₓ = 370 nm, | E_{1%} = 1346 |
| | λₘₐₓ = 356 nm (épaulement), | E_{1%} = 1031. |

### Deuxième étape : Préparation du composé de l'exemple 1 :

A une solution du produit précédent (0,508 g, 1,5.1x10⁻³ mole) et de catalyseur (complexe à 3-3.5% poids de Pt dans du cyclovinylméthylsiloxane de Hüls Petrarch PC085 : 100 µl) dans 2 ml de toluène sec porté à 80°C, on ajoute goutte à goutte en 10 minutes, 0,371 g (1,67x10⁻³ mole) d'heptaméthyltrisiloxane. On laisse à cette température pendant 6 heures. On concentre le mélange réactionnel. On reprend dans le dichlorométhane et passe cette solution sur un lit de Célite. L'huile jaune pâle obtenue est chromatographiée sur colonne de silice (éluant : Heptane/EtOAc 65 :35). On obtient ainsi 0,45 g (Rendement : 53 %) les fractions propres du dérivé de l'exemple 1 sous forme d'une huile jaune pâle qui cristallise dans le temps :

| | | |
|---|---|---|
| UV (Ethanol) : | λₘₐₓ = 372 nm, | E_{1%} = 1154 |
| | λₘₐₓ = 356 nm (épaulement), | E_{1%} = 773. |

mole) et on porte le mélange au reflux pendant 5 heures. On évapore à sec sous pression réduite l'éthanol. L'huile orangée obtenue est purifiée par une colonne chromatographique de silice (éluant : AcOEt / Heptane 50 :50 puis gradient jusque 30 :70. On récupère 1,68 g de fractions propres (Rendement: 71%) d'éthyl-5-[méthyl(prop-2-ynyl)amino]-2-(phénylsulfonyl)penta-2,4-diènoate sous forme d'une huile jaune pâle:

| | | |
|---|---|---|
| UV (CH₂Cl₂) : | λₘₐₓ = 366 nm, | E_{1%} = 1367. |
| | λₘₐₓ = 358 nm (épaulement), | E_{1%} = 1298. |

### Deuxième étape : Préparation du composé de l'exemple 3 :

A une solution du produit précédent (0,562 g, 1,69x10⁻³ mole) et de catalyseur (complexe à 3-3.5% poids de Pt dans du cyclovinylméthylsiloxane de Hüls Petrarch PC085 : 60 µl) dans 2 ml de toluène sec porté à 80°C, on ajoute goutte à goutte en 10 minutes, 0,413 g (1,86x10⁻³ mole) d'heptaméthyltrisiloxane. On laisse à cette température pendant 6 heures. On concentre le mélange réactionnel. On reprend dans le dichlorométhane et passe cette solution sur un lit de Célite. L'huile jaune pâle obtenue est chromatographiée sur colonne de silice (éluant : CH₂Cl₂ ). On obtient ainsi 0,35 g (Rendement : 37 %) les fractions propres du dérivé de l'exemple 3 sous forme d'une huile jaune orangée qui cristallise dans le temps et dans le rapport 25 :75 déterminé par ¹H RMN :

| | | |
|---|---|---|
| UV (Ethanol) : | λₘₐₓ = 366 nm, | E_{1%} = 1058 |
| | λₘₐₓ = 356 nm (épaulement), | E_{1%} = 705. |

### EXEMPLE 4 :

### Préparation du mélange de composés (j)

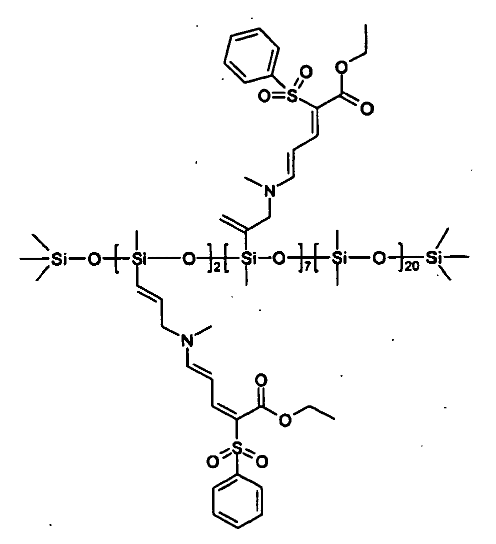

A une solution de d'éthyl-5-[méthyl(prop-2-ynyl)amino]-2-(phénylsulfonyl)penta-2,4-diènoate (0,8 g, 2,41x10⁻³ mole) obtenu à la première étape de l'exemple 3 et de

### EXEMPLE 2 :

### Préparation du composé (h)

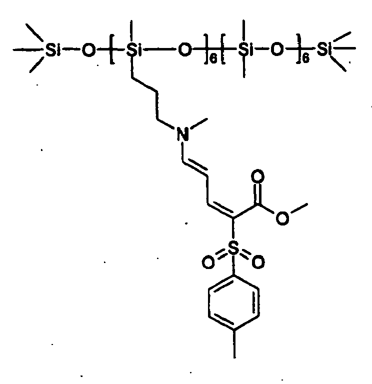

A une solution de méthyl-5-[allyl(méthyl)amino]-2-[(4-méthylphényl)sulfonyl]penta-2,4-diènoate (0,7 g, 2,1x10⁻³ mole) obtenu à la première étape de l'exemple 1 et de catalyseur (complexe à 3-3.5% poids de Pt dans du cyclovinylméthylsiloxane de Hüls Petrarch PC085 : 80 µl) dans 2 ml de toluène sec porté à 80°C, on ajoute goutte à goutte en 10 minutes, 0,325 g (2 méq SiH) de méthylhydro (50-55%) diméthylsiloxane (45-50%) copolymer (PS122.5 de Petrarch). On laisse à cette température pendant 6 heures. On concentre le mélange réactionnel. On reprend dans le dichlorométhane et passe cette solution sur un lit de Célite. L'huile jaune pâle obtenue est chromatographiée sur colonne de silice (éluant : CH₂Cl₂). On obtient ainsi 0,92 g des fractions propres du dérivé de l'exemple 2 sous forme d'une huile jaune pâle visqueuse :

| | | |
|---|---|---|
| UV (Ethanol) : | λₘₐₓ = 371 nm, | E_{1%} = 728. |

### EXEMPLE 3 :

### Préparation du mélange de composés (i)

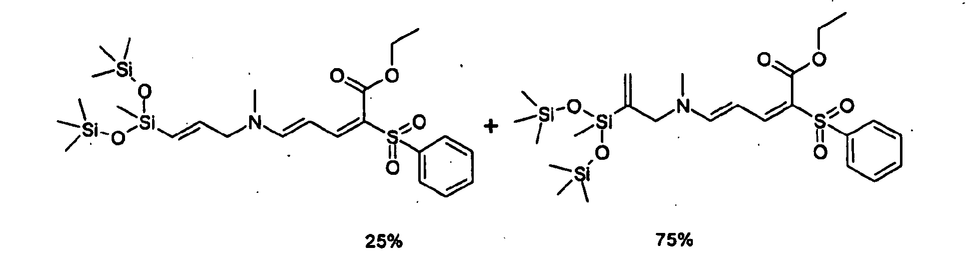

### Première étape : Préparation de l'éthyl-5-[méthyl(prop-2-ynyl)amino]-2-(phénylsulfonyl)penta-2.4-diènoate :

On chauffe à 85-90°C dans 5 ml d'anhydride acétique du 3-anilinoacroléine aniline (1,5 g, 6,75x10⁻³ mole) et du phénylsulphonylacetate d'éthyle (1,848 g, 8,1x10⁻³ mole) pendant 3 heures. On évapore à sec sous pression réduite l'anhydride acétique. L'huile obtenue est reprise dans 5 ml d'éthanol. On y ajoute de la N-méthyl propargylamine (1,22 ml, 0,0146 catalyseur (complexe à 3-3.5% poids de Pt dans du cyclovinylméthylsiloxane de Hüls Petrarch PC085 : 110 µl) dans 2 ml de toluène sec porté à 80°C, on ajoute goutte à goutte en 10 minutes, 1,24 g (2,3 méq SiH) de méthylhydro (30-35%) diméthylsiloxane (65-70%) copolymer (Huile PDMS à SiH 628V14 de Rhône-Poulenc). On laisse à cette température pendant 6 heures. On concentre le mélange réactionnel. On reprend dans le dichlorométhane et passe cette solution sur un lit de Célite. L'huile jaune pâle obtenue est chromatographiée sur colonne de silice (éluant : CH₂Cl₂). On obtient ainsi 1,82 g des fractions propres du dérivé de l'exemple 4 sous forme d'une huile jaune pâle visqueuse :

| | | |
|---|---|---|
| UV (Ethanol) : | λₘₐₓ = 366 nm, | E_{1%} = 270. |

### EXEMPLES DE FORMULATION

### EXEMPLE A :

- Mélange mono/distéarate de glycérol/stéarate de 1,0 g polyéthylèneglycol 100 OE (ARLACEL 165 FL-ICI)
- Alcool cétylique 0,5 g
- Acide stéarique d'huile de palme 2.5 g (STEARINE TP-STEARINERIE DUBOIS)
- Polydiméthylsiloxane 0.5 g (DOW CORNING 200 FLUID-DOW CORNING)
- Benzoate d'alcools en C₁₂/C₁₅ 20 g (WITCONOL TN- WITCO)
- Composé de l'exemple 1 2 g
- Glycérine 5.0 g
- Phosphate d'alcool hexadécylique, sel de potassium 1.0 g (AMPHISOL K-HOFFMANN LA ROCHE)
- Acide polyacrylique 0.3 g (SYNTHALEN K- 3V)
- Hydroxypropylméthylcellusose 0.1 g ( METHOCEL F4M- DOW CHEMICAL)
- Cyclopentadiméthylsiloxane 2.0 g (DC245-DOW CORNING)
- Triéthanolamine 0.8 g
- Conservateurs qs
- Eau déminéralisée qsp 100 g

### EXEMPLE B :

- Mélange d'alcool cétylstéarylique 7.0 g et d'alcool cétylstéarylique oxyéthyléné
   à 33 OE (80/20) (SINNOWAX AO-HENKEL)
- Mélange de mono- et de di-stéarate de glycérol 2.0 g (CERASYNT SD-V de ISP)
- Alcool cétylique 1.5 g
- Polydiméthylsiloxane 1.5 g (DOW CORNING 200 FLUID-DOW CORNING)
- Benzoate d'alcools en C₁₂/C₁₅ 8.0 g (WITCONOL TN- WITCO)
- Huile de vaseline 10.0 g
- Composé de l'exemple 2 2.0 g
- Glycérine 10.0 g
- Conservateurs qs
- Eau déminéralisée qsp 100 g

## Revendications

1. Composé silanique, siloxanique ou polysiloxanique de sulfone mérocyanine **choisi** parmi
(i) ceux répondant à l'une ou à l'autre des formules (1), (2) ou (3) suivantes, qui peuvent être présents sous les formes isomériques E, E-, E, Z-, ou Z,Z- ; 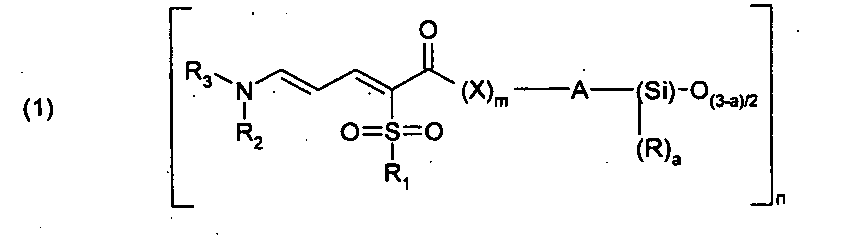 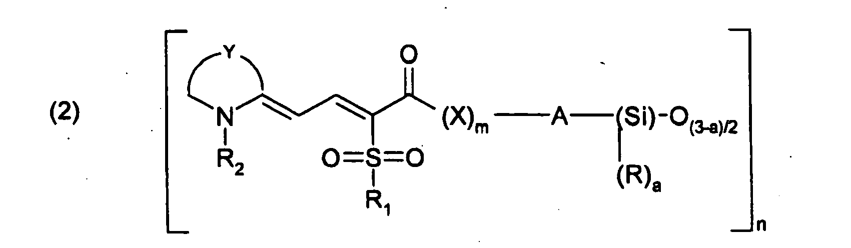 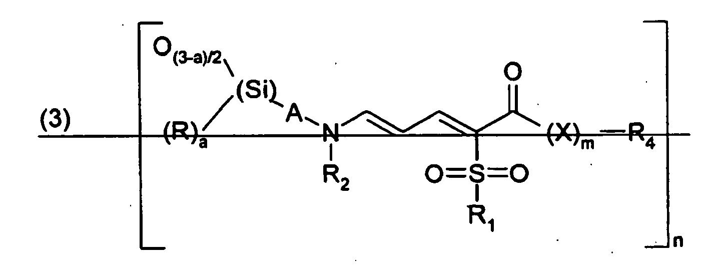 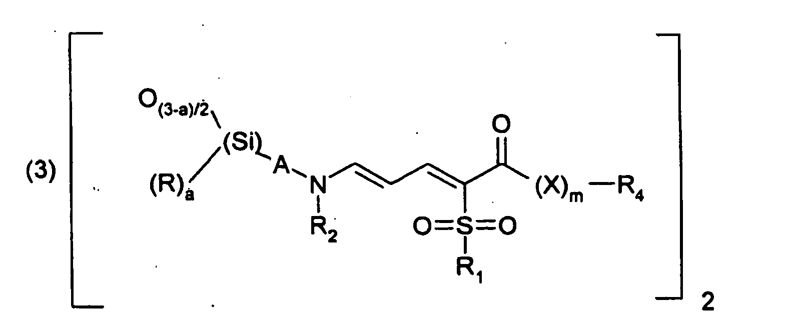 dans laquelle :
m = 0 ou 1,
X représente -O-, -NR₅-, -SO₂NH-, avec R₅ qui représente l'hydrogène ou un radical alkyle en C₁-C₅ linéaire ou ramifié,
Y est un radical divalent alkyle en C₁-C₅ éventuellement substitué par des radicaux alkyles en C₁-C₄ et/ou contenant des atomes -O-, -S-, ou par un groupe -NR₁,
R, identiques ou différents représentent un radical alkyle en C₁-C₂₀, linéaire ou ramifié et éventuellement halogéné, un radical aryle en C₆-C₁₂ ou un groupe alkoxy en C₁-C₁₀, a = 0 à 3,
R₁ représente un radical alkyle en C₁-C₂₀, linéaire ou ramifié et éventuellement halogéné ou un radical aryle en C₆-C₁₂,
R₂, R₃ , identiques ou différents, représentent H, un radical alkyle en C₁-C₂₄ linéaire ou ramifié, un radical cycloalkyle en C₃-C₁₀, aryle en C₆-C₂₀ substitué par des groupements alkyle ou alcoxy, et R₂, R₃ ensembles avec l'atome d'azote peuvent former un cycle en C₄-C₆ éventuellement interrompu par des atomes d'oxygène ou -NH-, avec le fait que R₂, R₃ ne peuvent ensemble représenter un atome d'hydrogène,
R₄ est un groupe alkyle divalent
A est un radical divalent choisi parmi méthylène, éthylène ou un groupe répondant à l'une des formules (4) , (5) ou (6) suivantes : 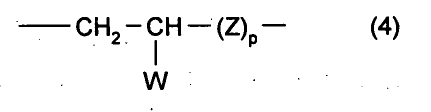
-CH=CH-(Z)ₚ- (5) 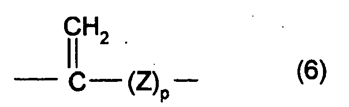
dans lesquelles :
Z est un radical alkylène en C₁-C₆ linéaire ou ramifié, saturé ou insaturé, éventuellement substitué par un radical hydroxyle ou un radical alkyle en C₁-C₈, linéaire ou ramifié, saturé ou insaturé,
W représente un atome d'hydrogène ; un radical hydroxyle ou un radical alkyle en C₁-C₈, linéaire ou ramifié, saturé ou insaturé,
p est 0 ou 1,
n = 1 à 4 et dans le cas où n = 2, R₁, R₂ ou R₃ est un groupe alkyle divalent ou bien R₂ et R₃ ensembles avec l'atome d'azote peuvent former un cycle en C₄-C₆ éventuellement interrompu par des atomes d'oxygène ou -NH- ;
ledit composé pouvant comporter, en plus des unités de formule -A-(Si)(R)ₐ(O)_{(3-a)/2}, des unités de formule (R)_{b}-(Si)(O)_{(4-b)/2} dans lesquelles :
R a la même signification que dans les formules (1) à (3),
b = 1, 2 ou 3 ;
(ii) ceux choisis parmi les composés suivants :

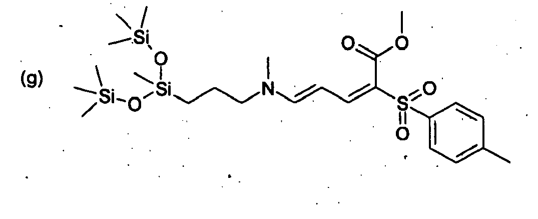

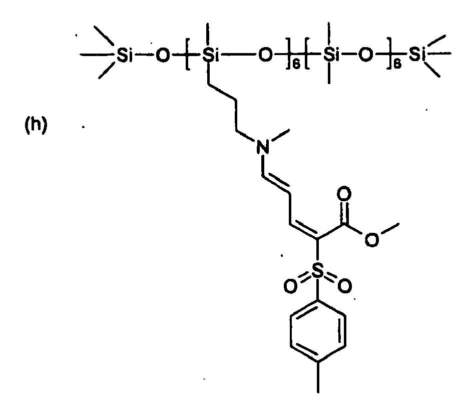

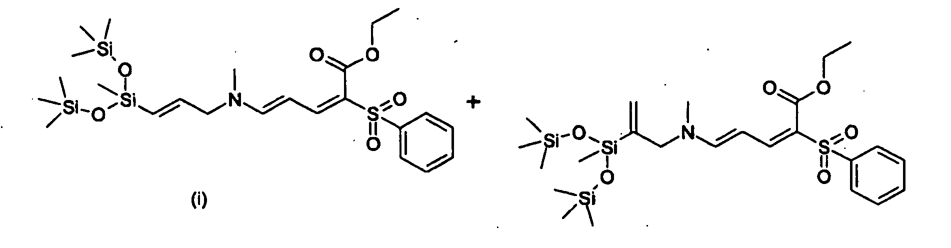

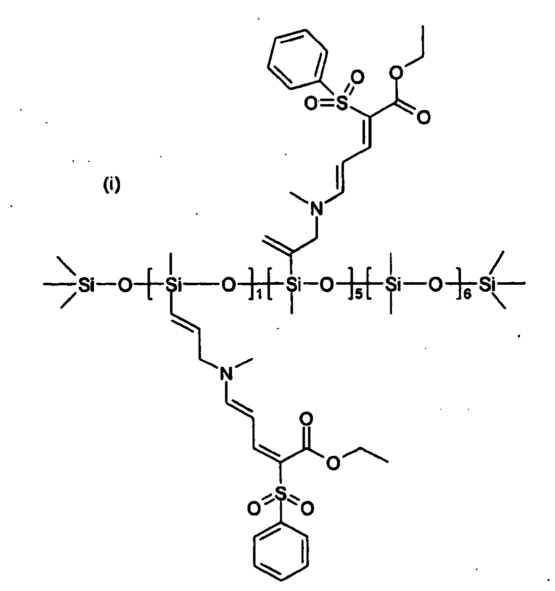

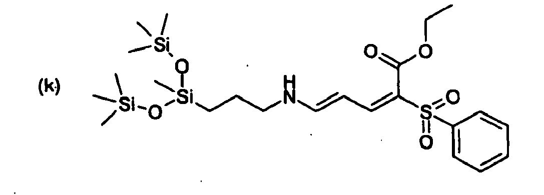

2. Composé selon la revendication 1, où le groupement -(Si)(R)ₐ(O)_{(3-a)/2} est représenté par l'une des formules (7), (8) ou (9) suivantes : 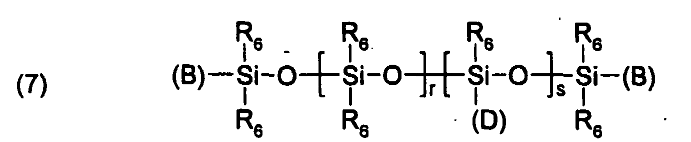 ou 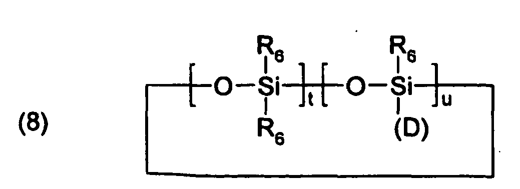
(9) (D)-Si(R₆)₃
dans lesquelles :
(D) est liant entre la chaîne siliconée au groupement A des chromophores des formules (1) à (3),
R₆, identiques ou différents, sont choisis parmi les radicaux alkyles linéaires ou ramifiés en C₁-C₃₀, phényle, 3,3,3-trifluoropropyle et triméthylsilyloxy, au moins 80% en nombre des radicaux R₆ étant méthyle,
(B), identiques ou différents sont choisis parmi les radicaux R₆ et le radical A
r est un nombre entier compris entre 0 et 200 inclusivement, et s est un nombre entier
compris entre 0 et 50 inclusivement, et si s = 0, au moins l'un des deux symboles (B) désigne A,
u est un nombre entier compris entre 1 et 10 inclusivement, et t est un nombre entier compris entre 0 et 10 inclusivement, étant entendu que t + u est égal ou supérieur à 3.

3. Composé selon la revendication 2, **caractérisé par le fait qu'**il désigne un diorganosiloxane linéaire ou cyclique de formule (7) ou (8) présentant au moins l'une, et encore plus préférentiellement l'ensemble, des caractéristiques suivantes :
- R₈ est méthyle,
- B est méthyle.

4. Composé selon l'une quelconque des revendications 1 à 3, **caractérisé par le fait qu'**il désigne un composé de formule (1) choisi parmi ceux de formules (a) à (e) suivantes :

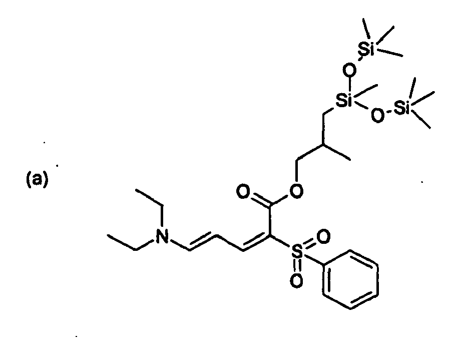

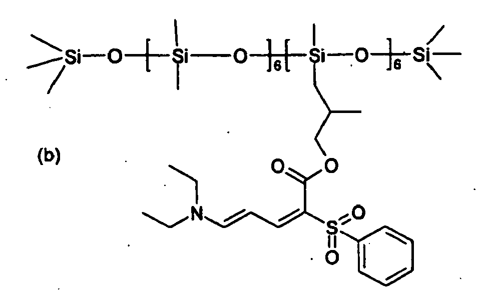

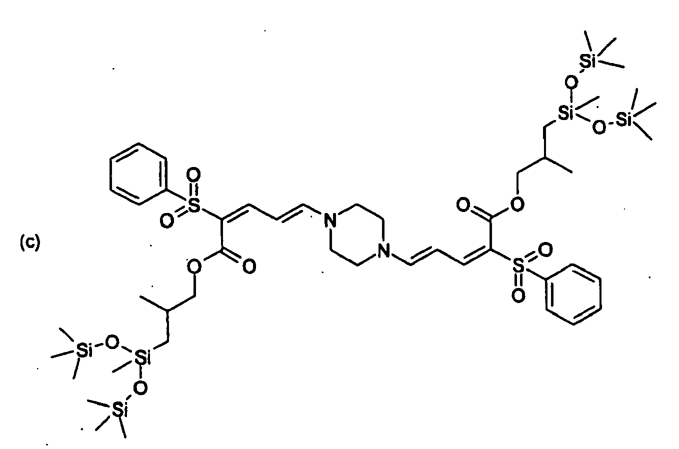

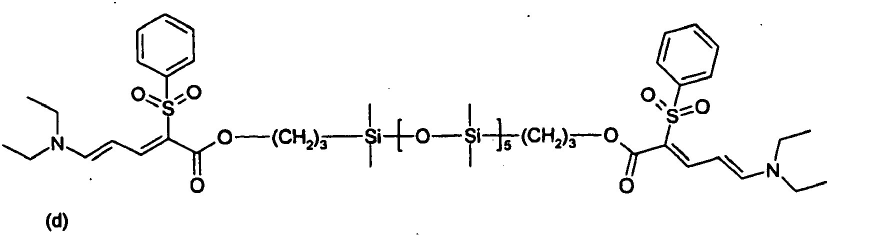

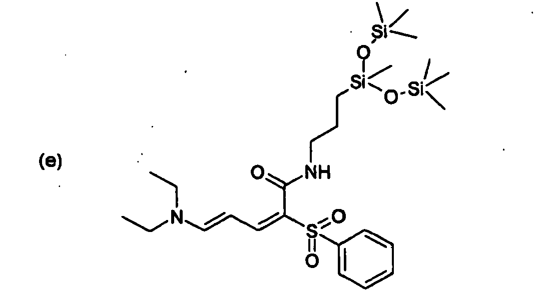

5. Composé selon l'une quelconque des revendications 1 à 3, **caractérisé par le fait qu'**il désigne le composé de formule (2) tel celui de formule (f) suivant :

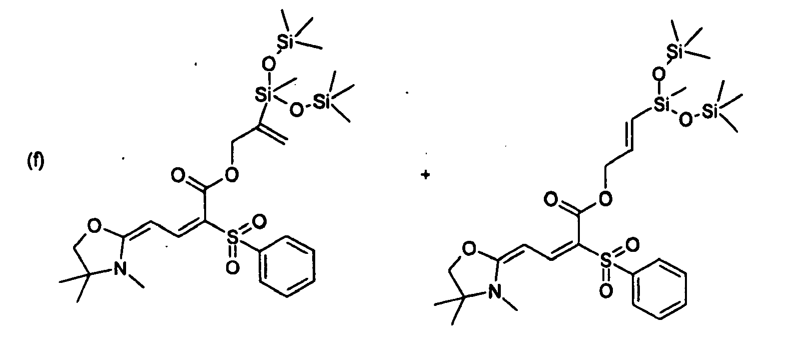

6. Composition cosmétique ou dermatologique, contenant dans un milieu physiologiquement acceptable au moins un composé silanique, siloxanique ou polysiloxanique de sulfone mérocyanine tel que défini dans l'une des revendications précédentes.

7. Composition selon la revendication 6, où le ou les composés silaniques, siloxaniques ou polysiloxaniques de sulfone mérocyanine sont présents dans des proportions allant de 0,01 % à 20 % en poids, de préférence de 0,1 % à 10 % en poids, par rapport au poids total de la composition.

8. Composition selon l'une quelconque des revendications 1 à 7, **caractérisée par le fait qu'**elle contient en plus d'autres agents photoprotecteurs organiques ou inorganiques actifs dans l'UV-A et/ou l'UV-B hydrosolubles ou liposolubles ou bien insolubles dans les solvants cosmétiques couramment utilisés.

9. Composition selon la revendication 8, où les agents photoprotecteurs organiques complémentaires sont choisis parmi les anthranilates ; les dérivés cinnamiques ; les dérivés salicyliques, les dérivés du camphre ; les dérivés de la benzophénone ; les dérivés de β,β-diphénylacrylate ; les dérivés de triazine ; les dérivés de benzotriazole ; les dérivés de benzalmalonate ; les dérivés de benzimidazole ; les imidazolines ; les dérivés bis-benzoazolyle ; les dérivés de l'acide p-aminobenzoïque (PABA) ; les dérivés de méthylène bis-(hydroxyphényl benzotriazole) ; les dérivés de benzoxazole ; les polymères filtres et silicones filtres ; les dimères dérivés d'α-alkylstyrène ; les 4,4-diarylbutadiènes et leurs mélanges.

10. Composition selon la revendication **9**, **caractérisée par le fait que** le ou les filtres UV organiques sont choisis parmi les composés suivants :
Ethylhexyl Salicylate,
Homosalate,
Ethylhexyl Methoxycinnamate
Octocrylene,
Phenylbenzimidazole Sulfonic Acid,
Disodium Phenyl Dibenzimidazole Tetra-sulfonate,
Benzophenone-3,
Benzophenone-4,
Benzophenone-5,
Diethylamino Hydroxybenzoyl Hexyl Benzoate.
4-Methylbenzylidene camphor,
Terephthalylidene Dicamphor Sulfonic Acid,
Bis-Ethylhexyloxyphenol Methoxyphenyl Triazine
Ethylhexyl Triazone,
Diethylhexyl Butamido Triazone,
Methylene bis-Benzotriazolyl Tetramethylbutylphénol
Drometrizole Trisiloxane
Polysilicone-15
1,1-dicarboxy (2,2'-diméthyl-propyl)-4,4-diphénylbutadiene
2,4-bis-[5-1(diméthylpropyl)benzoxazol-2-yl-(4-phenyl)-imino]-6-(2-ethylhexyl)-imino-1,3,5-triazine
et leurs mélanges.

11. Composition selon la revendication 8, **caractérisée par le fait que** les agents photoprotecteurs inorganiques complémentaires sont des pigments ou des nanopigments d'oxydes métalliques, traités ou non.

12. Composition selon la revendication 11, **caractérisée par le fait que** lesdits pigments ou nanopigments sont choisis parmi les oxydes de titane, de zinc, de fer, de zirconium, de cérium et leurs mélanges, traités ou non.

13. Composition selon l'une quelconque des revendications 6 à 12, **caractérisée par le fait qu'**elle comprend en outre au moins un agent de bronzage et/ou de brunissage artificiel de la peau.

14. Composition selon l'une quelconque des revendications 6 à 13, **caractérisée par le fait qu'**elle comprend en outre au moins un adjuvant choisi parmi les corps gras, les solvants organiques, les épaississants ioniques ou non ioniques, hydrophiles ou lipophiles, les adoucissants, les humectants, les opacifiants, les stabilisants, les émollients, les silicones, les agents anti-mousse, les parfums, les conservateurs, les tensioactifs anioniques, cationiques, non-ioniques, zwitterioniques ou amphotères, des actifs, les charges, les polymères, les propulseurs, les agents alcalinisants ou acidifiants

15. Utilisation d'une composition telles que définie dans l'une quelconque des revendications 6 à 14 pour la fabrication de produits pour le traitement cosmétique de la peau, des lèvres, des ongles, des cheveux, des cils, sourcils et/ou du cuir chevelu

16. Utilisation d'une composition telles que définie dans l'une quelconque des revendications 6 à 14 pour la fabrication de produits de soin de la peau, des lèvres, des ongles, des cheveux et/ou du cuir chevelu.

17. Utilisation d'une composition telles que définie dans l'une quelconque des revendications 6 à 14 pour la fabrication de produits de maquillage.

18. Utilisation d'un composé silanique, siloxanique ou polysiloxanique de sulfone mérocyanine tel que défini dans les revendications 1 à 6 dans une composition cosmétique ou dermatologique comme agent filtrant les radiations UV.

19. Utilisation d'un composé silanique, siloxanique ou polysiloxanique de sulfone mérocyanine tel que défini dans les revendications 1 à 6 comme agent de contrôle de la variation de la couleur de la peau due aux rayonnements UV.

20. Utilisation d'un composé silanique, siloxanique ou polysiloxanique de sulfone mérocyanine tel que défini dans les revendications 1 à 6 comme agent photostabilisant de polymères synthétiques tels que les matières plastiques ou de verres en particulier des verres de lunettes ou des lentilles de contact.

## Claims

1. Silane, siloxane or polysiloxane merocyanine sulphone compound chosen from
(i) those having one or other of formulae (1), (2) or (3) below and which may be present in the isomeric E,E-, E,Z- or Z,Z- forms: 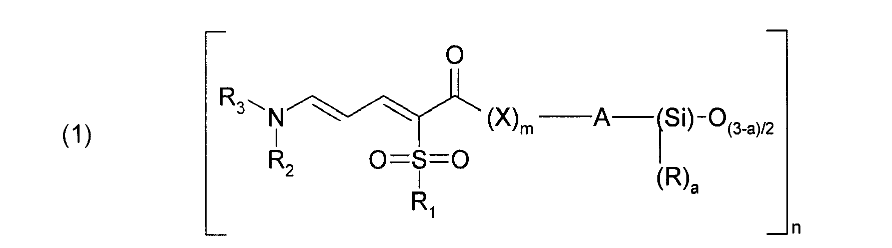 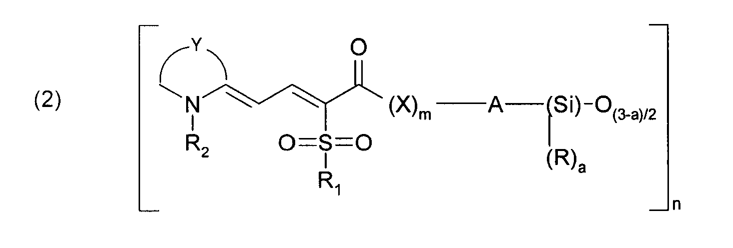 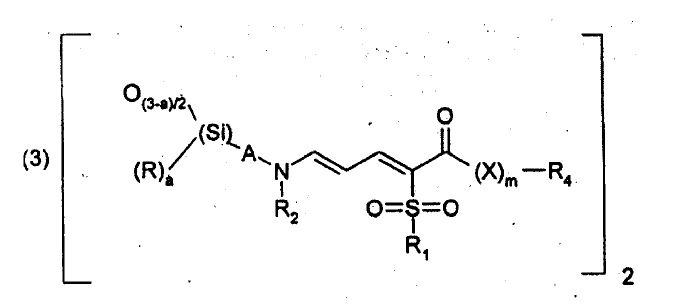 in which:
m = 0 or 1;
X represents -O-, -NR₅-, -SO₂NH-, where R₅ represents hydrogen or a linear or branched C₁-C₅ alkyl radical;
Y is a divalent C₁-C₅ alkyl radical optionally substituted with C₁-C₄ alkyl radicals and/or containing -O-, -S- atoms or with a -NR₁ group;
R, which may be identical or different, represent a linear or branched and optionally halogenated C₁-C₂₀ alkyl radical, a C₆-C₁₂ aryl radical or a C₁-C₁₀ alkoxy group;
a = 0 to 3;
R₁ represents a linear or branched and optionally halogenated C₁-C₂₀ alkyl radical or a C₆-C₁₂ aryl radical;
R₂, R₃, which may be identical or different, represent H, a linear or branched C₁-C₂₄ alkyl radical, a C₃-C₁₀ cycloalkyl radical or a C₆-C₂₀ aryl radical substituted with alkyl or alkoxy groups, and R₂, R₃ together with the nitrogen atom may form a C₄-C₆ cycle optionally interrupted by oxygen atoms or -NH-, wherein R₂, R₃ cannot together represent a hydrogen atom;
R₄ is a divalent alkyl group;
A is a divalent radical selected from methylene, ethylene or a group having one of formulae (4), (5) or (6) below: 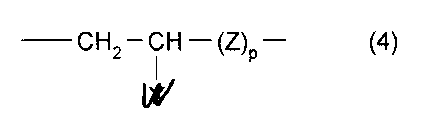
-CH=CH-(Z)ₚ- (5) 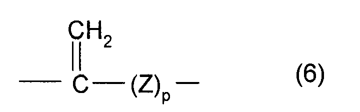
in which:
Z is a linear or branched, saturated or unsaturated C₁-C₆ alkylene radical optionally substituted with a hydroxyl radical, or a linear or branched, saturated or unsaturated C₁-C₈ alkyl radical;
W represents a hydrogen atom; a hydroxyl radical or a linear or branched, saturated or unsaturated C₁-C₈ alkyl radical;
p is 0 or 1;
n = 1 to 4 and when n = 2, R₁, R₂ or R₃ is a divalent alkyl group or else R₂ and R₃ together with the nitrogen atom may form a C₄-C₆ cycle optionally interrupted by oxygen atoms or -NH-;
said compound possibly containing, in addiction of the units of formula -A-(Si)(R)ₐ(O)_{(3-a)/2}, units of formula (R)_{b}-(Si) (O)_{(4-b)/2} in which:
R has the meaning given in formulae (1) to (3);
b = 1, 2 or 3;
(ii) those chosen from the following compounds:

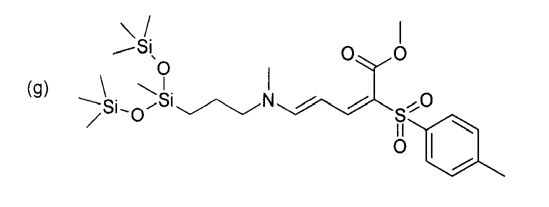

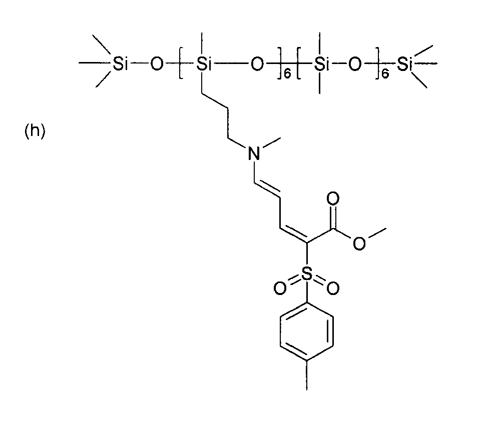

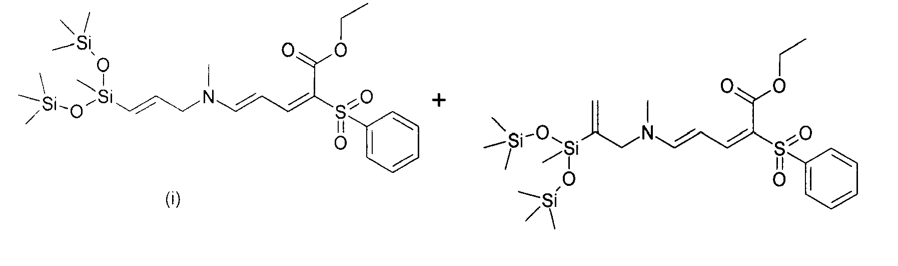

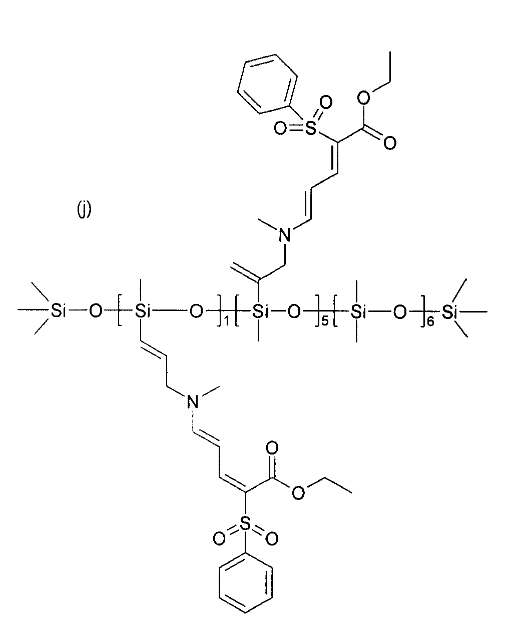

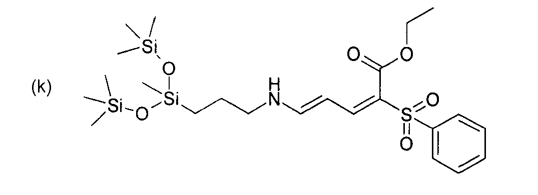

2. Compound according to Claim 1, in which the group -(Si)(R)ₐ(O)_{(3-a)/2} is represented by one of formulae (7), (8) or (9) below: 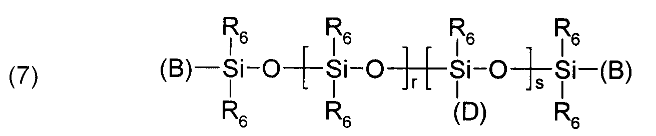 or 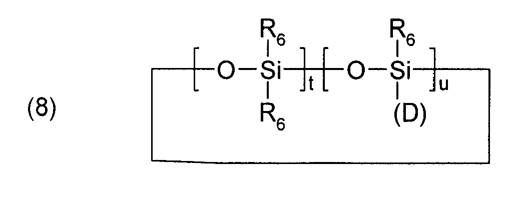
(9) (D)-Si(R₆)₃
in which:
(D) is a linkage between the silicone chain and group A of the chromophores of formulae (1) to (3);
R₆, which may be identical or different, are selected from linear or branched C₁-C₃₀ alkyl, phenyl, 3,3,3-trifluoropropyl and trimethylsilyloxy radicals, at least 80% in number of radicals R₆ being methyl;
(B), which may be identical or different, are selected from radicals R₆ and radical A;
r is a whole number in the range 0 to 200 inclusive and s is a whole number in the range 0 to 50 inclusive, and if s = 0, at least one of the two symbols (B) designates A;
u is a whole number in the range 1 to 10 inclusive and t is a whole number in the range 0 to 10 inclusive, it being understood that t + u is 3 or more.

3. Compound according to Claim 2, **characterized in that** it designates a linear or cyclic diorganosiloxane of formula (7) or (8) having at least one, and more preferably all, of the following characteristics:
- R₆ is methyl,
- B is methyl.

4. Compound according to any one of Claims 1 to 3, **characterized in that** it designates a compound of formula (1) selected from formulae (a) to (e) below:

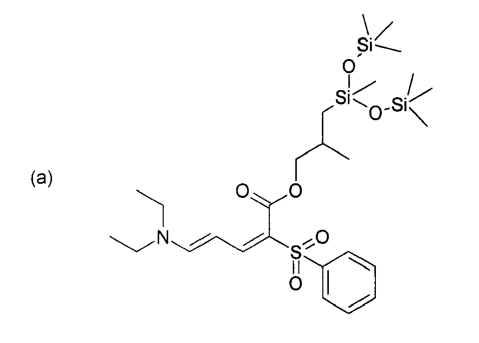

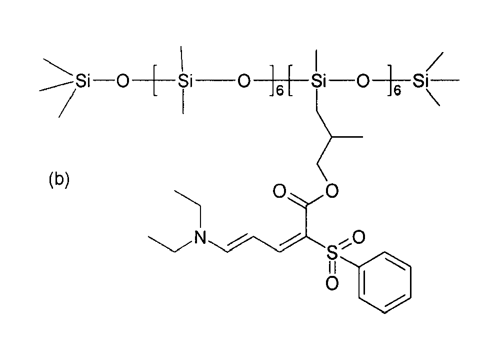

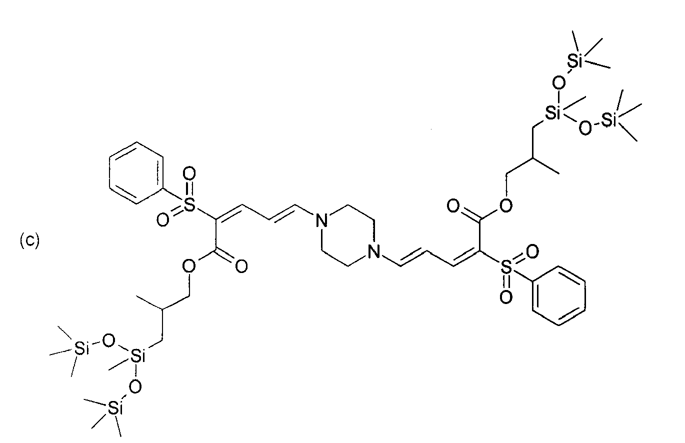

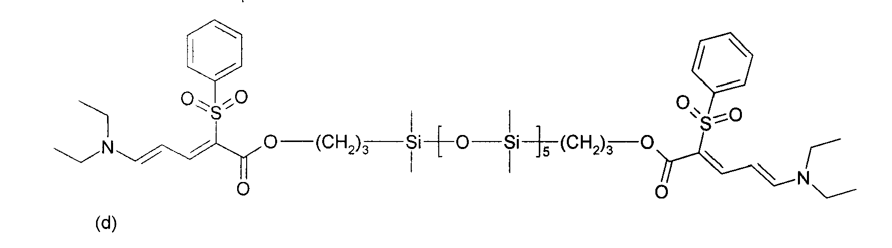

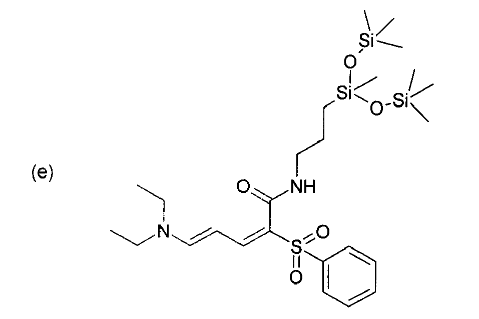

5. Compound according to any one of Claims 1 to 3, **characterized in that** it designates the compound of formula (2) as defined in formula (f) below:

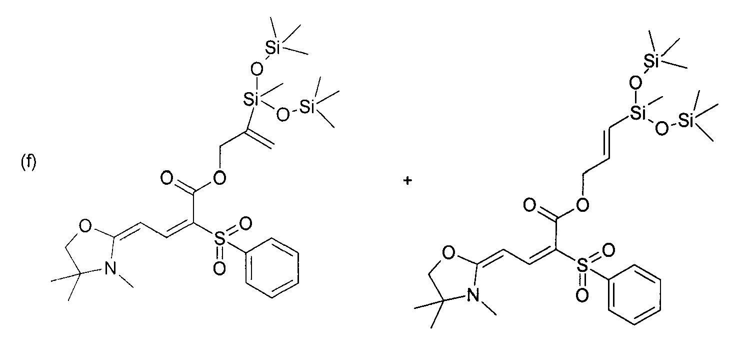

6. Cosmetic or dermatological composition containing, in a physiologically acceptable medium, at least one silane, siloxane or polysiloxane merocyanine sulphone compound as defined in one of the preceding claims.

7. Composition according to Claim 6, in which the silane, siloxane or polysiloxane merocyanine sulphone compound or compounds are present in proportions of 0.01% to 20% by weight, preferably 0.1% to 10% by weight with respect to the total composition weight.

8. Composition according to any one of Claims 1 to 7, **characterized in that** it further contains other organic or inorganic photoprotective agents which are active in the UV-A and/or UV-B, which are hydrosoluble or liposoluble or even insoluble in cosmetic solvents in current use.

9. Composition according to Claim 8, in which the complementary organic photoprotective agents are selected from anthranilates; cinnamic derivatives; salicylic derivatives, camphor derivatives; benzophenone derivatives; β,β-diphenylacrylate derivatives; triazine derivatives; benzotriazole derivatives; benzalmalonate derivatives; benzimidazole derivatives; imidazolines; bis-benzoazolyl derivatives; p-aminobenzoic acid (PABA) derivatives; methylene bis-(hydroxyphenyl benzotriazole) derivatives; benzoxazole derivatives; polymer filters and silicone filters; dimers derived from α-alkylstyrene; 4,4-diarylbutadienes and mixtures thereof.

10. Composition according to Claim 9, **characterized in that** the organic UV filter or filters are selected from the following compounds:
Ethylhexyl Salicylate;
Homosalate;
Ethylhexyl Methoxycinnamate;
Octocrylene;
Phenylbenzimidazole Sulphonic Acid;
Disodium Phenyl Dibenzimidazole Tetra-sulphonate;
Benzophenone-3;
Benzophenone-4;
Benzophenone-5;
Diethylamino Hydroxybenzoyl Hexyl Benzoate;
4-Methylbenzylidene camphor;
Terephthalylidene Dicamphor Sulphonic Acid;
Bis-Ethylhexyloxyphenol Methoxyphenyl Triazine;
Ethylhexyl Triazone;
Diethylhexyl Butamido Triazone;
Methylene bis-Benzotriazolyl Tetramethylbutylphenol;
Drometrizole Trisiloxane;
Polysilicone-15;
1,1-dicarboxy (2,2'-dimethylpropyl)-4,4-diphenylbutadiene;
2,4-bis-[5-1(dimethylpropyl)benzoxazol-2-yl-(4-phenyl)imino]-6-(2-ethylhexyl)imino-1,3,5-triazine;
and mixtures thereof.

11. Composition according to Claim 8, **characterized in that** the complementary inorganic photoprotective agents are treated or untreated pigments or nanopigments of metal oxides.

12. Composition according to Claim 11, **characterized in that** said pigments or nanopigments are selected from treated or untreated oxides of titanium, zinc, iron, zirconium, cerium and mixtures thereof.

13. Composition according to any one of Claims 6 to 12, **characterized in that** it further comprises at least one artificial skin tanning and/or browning agent.

14. Composition according to any one of Claims 6 to 13, **characterized in that** it further comprises at least one adjuvant selected from fats, organic solvents, ionic or non-ionic, hydrophilic or lipophilic thickening agents, softeners, moisturizers, opacifying agents, stabilizers, emollients, silicones, anti-foaming agents, fragrances, preservatives, anionic, cationic, non-ionic, zwitterionic or amphoteric surfactants, active ingredients, fillers, polymers, propellants or alkalinizing or acidifying agents.

15. Use of a composition as defined in any one of Claims 6 to 14 for the manufacture of products for the cosmetic treatment of the skin, lips, nails, hair, eyelashes, eyebrows and/or scalp.

16. Use of a composition as defined in any one of Claims 6 to 14 for the manufacture of products for the care of the skin, lips, nails, hair and/or scalp.

17. Use of a composition as defined in any one of Claims 6 to 14 for the manufacture of make-up products.

18. Use of a silane, siloxane or polysiloxane merocyanine sulphone compound as defined in Claims 1 to 6 in a cosmetic or dermatological composition as a UV radiation filter.

19. Use of a silane, siloxane or polysiloxane merocyanine sulphone compound as defined in Claims 1 to 6 as an agent for controlling the variation in skin colour due to UV radiation.

20. Use of a silane, siloxane or polysiloxane merocyanine sulphone compound as defined in Claims 1 to 6 as an agent for photostabilizing synthetic polymers such as plastic materials or glass, in particular spectacle lenses or contact lenses.

## Patentansprüche

1. Silanmerocyaninsulfon, Siloxanmerocyaninsulfon oder Polysiloxanmerocyaninsulfon, das ausgewählt ist unter:
(i) solchen Verbindungen, die einer der folgenden Formeln (1), (2) oder (3) entsprechen, wobei sie in den isomeren Formen E,E-, E,Z- oder Z,Z- vorliegen können: 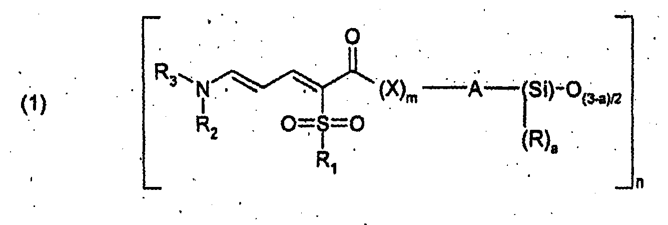 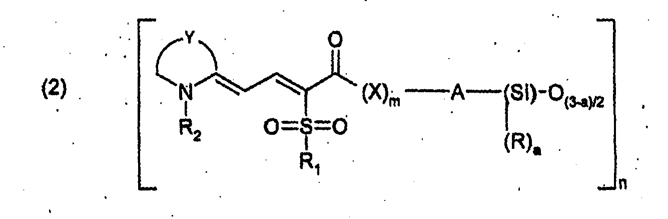 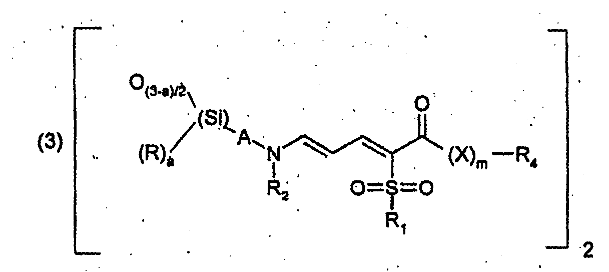 in der Formel:
m = 0 oder 1,
X bedeutet -O-, -NR₅-, -SO₂NH-, wobei R₅ Wasserstoff oder eine geradkettige oder verzweigte C₁₋₅-Alkylgruppe bedeutet,
Y ist eine zweiwertige C₁₋₅-Alkylgruppe, die gegebenenfalls mit C₁₋₄-Alkylgruppen substituiert ist und/oder Atome -O-, -S- oder eine Gruppe -NR₁ enthält,
die Gruppen R, die gleich oder verschieden sind, bedeuten eine geradkettige oder verzweigte, gegebenenfalls halogenierte C₁₋₂₀-Alkylgruppe, eine C₆₋₁₂-Arylgruppe oder eine C₁₋₁₀-Alkoxygruppe,
a = 0 bis 3,
R₁ bedeutet eine geradkettige oder verzweigte, gegebenenfalls halogenierte C₁₋₂₀-Alkylgruppe oder eine C₆₋₁₂-Arylgruppe, R₂ und R₃, die gleich oder verschieden sind, bedeuten H, eine geradkettige oder verzweigte C₁₋₂₄-Alkylgruppe, eine C₃₋₁₀-Cycloalkylgruppe, eine C₆₋₂₀-Arylgruppe, die mit Alkyl- oder Alkoxygruppen substituiert ist, und R₂ und R₃ können gemeinsam mit dem Stickstoffatom, an das sie gebunden sind,
einen C₄-₆-Ring bilden, der gegebenenfalls durch Sauerstoffatome oder -NH- unterbrochen ist, wobei R₂ und R₃ nicht gleichzeitig Wasserstoff bedeuten können,
R₄ ist eine zweiwertige Alkylgruppe,
A ist eine zweiwertige Gruppe, die unter Methylen, Ethylen oder einer Gruppe ausgewählt ist, die einer der folgenden Formeln (4), (5) oder (6) entspricht: 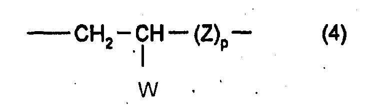
-CH=CH-(Z)ₚ- (5) 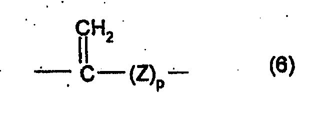
in den Formeln:
Z ist eine geradkettige oder verzweigte, gesättigte oder ungesättigte C₁₋₆-Alkylengruppe, die gegebenenfalls mit einer Hydroxygruppe oder einer geradkettigen oder verzweigten, gesättigten oder ungesättigten C₁₋₈-Alkylgruppe substituiert ist,
W bedeutet ein Wasserstoffatom; eine Hydroxygruppe oder eine geradkettige oder verzweigte, gesättigte oder ungesättigte C₁₋₈-Alkylgruppe,
p ist 0 oder 1,
n = 1 bis 4 und, wenn n = 2, bedeutet R₁, R₂ oder R₃ einer zweiwertige Alkylgruppe oder R₂ und R₃ können gemeinsam mit dem Stickstoffatom einen C₄₋₆-Ring bilden, der gegebenenfalls durch Sauerstoffatome oder -NH- unterbrochen ist,
wobei das Organosiloxan neben Einheiten der Formel
-A-(Si)(R)ₐ(O)_{(3-a)/2}
Einheiten der Formel (R)_{b}-(Si)(O)_{(4-b)/2} enthalten kann, wobei in den Formeln:
R hat die für die Formeln (1) bis (3) angegebenen Bedeutungen,
b = 1, 2 oder 3,
(ii) solchen, die unter den folgenden Verbindungen ausgewählt sind:

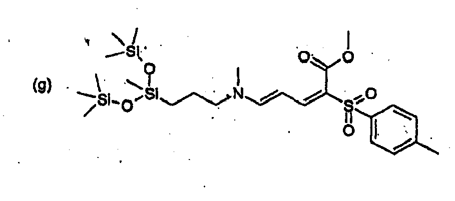

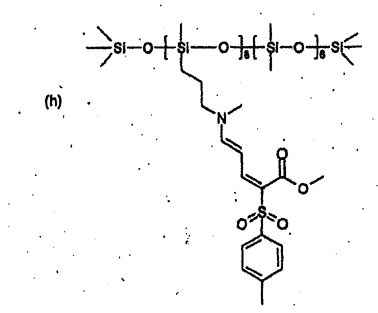

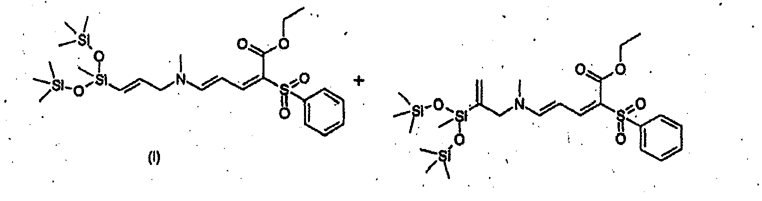

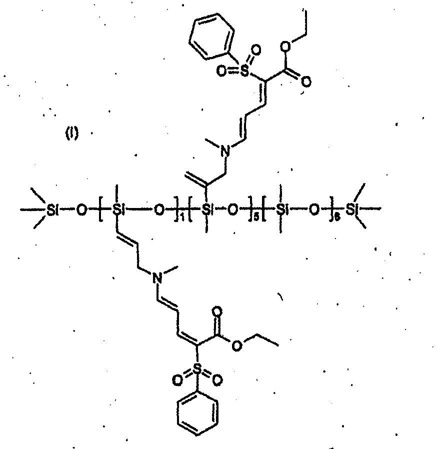

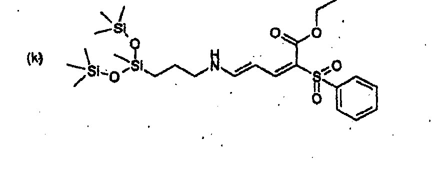

2. Verbindung nach Anspruch 1, wobei die Gruppierung -(Si)(R)ₐ(O)_{(3-a)/2} eine der folgenden Formeln (7), (8) oder (9) bedeutet: 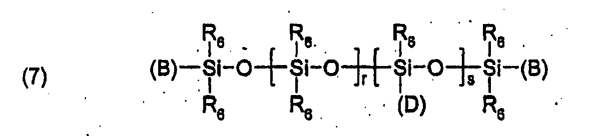 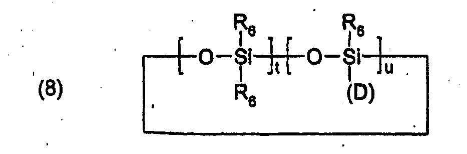
(9) (D)-Si(R₆)₃
in den Formeln:
(D) ist das Bindeglied zwischen der Siliconkette und der Gruppe A der Chromophoren der Formeln (1) bis (3),
die Gruppen R₆, die gleich oder verschieden sind, sind unter den geradkettigen oder verzweigten C₁₋₃₀-Alkylgruppen, Phenyl, 3,3,3-Trifluorpropyl und Trimethylsilyloxy ausgewählt, wobei mindestens 80 % der Anzahl der Gruppen R₆ Methyl bedeutet,
(B), die gleich oder verschieden sind, sind unter den Gruppen R₆ und der Gruppe A ausgewählt,
r ist eine ganze Zahl im Bereich von 0 bis einschließlich 200 und s ist eine ganze Zahl im Bereich von 0 bis einschließlich 50, wobei mindestens eines der beiden Symbole (B) A bedeutet, wenn s = 0,
u ist eine ganze Zahl im Bereich von 1 bis einschließlich 10 und t ist eine ganze Zahl im Bereich von 0 bis einschließlich 10, mit der Maßgabe, dass t + u größer oder gleich 3 ist.

3. Verbindung nach Anspruch 2, **dadurch gekennzeichnet, dass** sie ein lineares oder cyclisches Diorganosiloxan der Formel (7) oder (8) bedeutet, das mindestens eines und vorzugsweise alle folgenden Merkmale aufweist:
- R₆ bedeutet Methyl,
- B ist Methyl.

4. Verbindung nach einem der Ansprüche 1 bis 3, **dadurch gekennzeichnet, dass** sie eine Verbindung der Formel (1) bedeutet, die unter den folgenden Formeln (a) bis (e) ausgewählt ist:

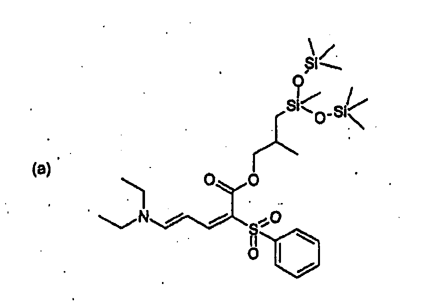

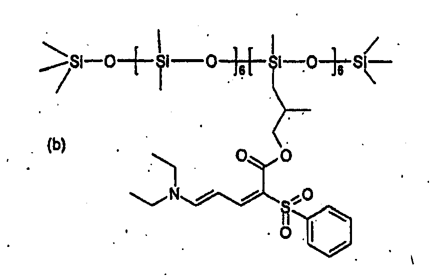

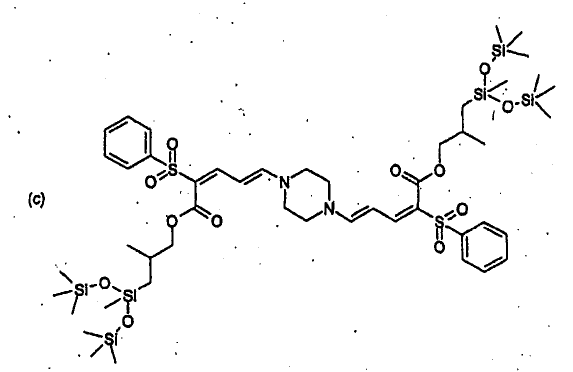

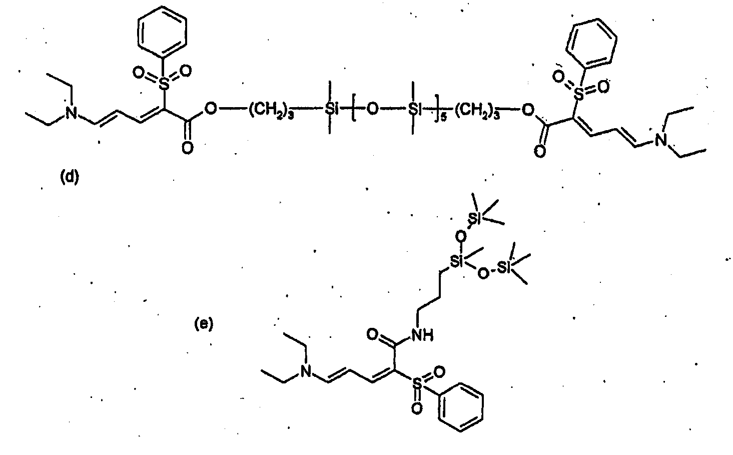

5. Verbindung nach einem der Ansprüche 1 bis 3, **dadurch gekennzeichnet, dass** sie eine Verbindung der Formel (2) bedeutet, wie die folgende Verbindung der folgenden Formel (f):

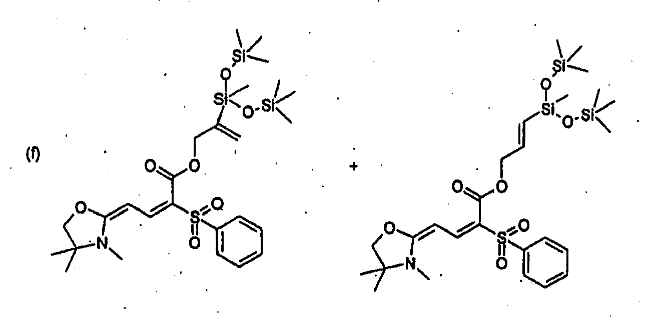

6. Kosmetische oder dermatologische Zusammensetzung, die in einem physiologisch akzeptablen Medium mindestens ein Silanmerocyaninsulfon, Siloxanmerocyaninsulfon oder Polysiloxanmerocyaninsulfon enthält, wie es in einem der vorhergehenden Ansprüche definiert ist.

7. Zusammensetzung nach Anspruch 6, wobei das oder die Silanmerocyaninsulfone, Siloxanmerocyaninsulfone oder Polysiloxanmerocyaninsulfone in Mengenanteilen von 0,01 bis 20 Gew.-% und vorzugsweise 0,1 bis 10 Gew.-%, bezogen auf das Gesamtgewicht der Zusammensetzung, enthalten sind.

8. Zusammensetzung nach einem der Ansprüche 1 bis 7, **dadurch gekennzeichnet, dass** sie ferner weitere organische oder anorganische, im UV-A- und/oder UV-B-Bereich wirksame Lichtschutzmittel enthält, die wasserlöslich oder fettlöslich oder in den gewöhnlich verwendeten kosmetischen Lösemitteln unlöslich sind.

9. Zusammensetzung nach Anspruch 8, wobei die ergänzenden organischen Lichtschutzmittel unter Anthranilaten; Zimtsäurederivaten; Salicylsäurederivaten; Campherderivaten; Benzophenonderivaten; β,β-Diphenyl-acrylatderivaten; Triazinderivaten; Benzotriazolderivaten; Benzalmalonatderivaten; Benzimidazolderivaten; Imidazolinen; Bisbenzoazolylderivaten; Derivaten der *p*-Aminobenzoesäure (PABA); Methylen-bis(hydroxyphenylbenzotriazol)derivaten; Benzoxazolderivaten; polymeren Filtern und Siliconfiltern; von α-Alkylstyrol abgeleiteten Dimeren; 4,4-Diarylbutadienen und deren Gemischen ausgewählt sind.

10. Zusammensetzung nach Anspruch 9, wobei der oder die ergänzenden organischen UV-Filter unter den folgenden Verbindungen ausgewählt sind:
Ethylhexyl Salicylate,
Homosalate,
Ethylhexyl Methoxycinnamate,
Octocrylene,
Phenylbenzimidazole Sulfonic Acid,
Disodium Phenyl Dibenzimidazole Tetra-sulfonate,
Benzophenone-3,
Benzophenone-4,
Benzophenone-5,
Diethylamino Hydroxybenzoyl Hexyl Benzoate,
4-Methylbenzylidene Camphor,
Terephthalylidene Dicamphor Sulfonic Acid,
Bis-Ethylhexyloxyphenol Methoxyphenyl Triazine,
Ethylhexyl Triazone,
Diethylhexyl Butamido Triazone,
Methylene bis-Benzotriazolyl Tetramethylbutylphenol,
Drometrizole Trisiloxane,
Polysilicone-15,
1, 1-Dicarboxy-(2,2'-dimethyl-propyl)-4,4-diphenylbutadien,
2,4-Bis[5-1(dimethylpropyl)benzoxazol-2-yl-(4-phenyl)-imino]-6-(2-ethylhexyl)-imino-1,3,5-triazin,
und deren Gemischen.

11. Zusammensetzung nach Anspruch 8, **dadurch gekennzeichnet, dass** die ergänzenden anorganischen Lichtschutzmittel unter den Pigmenten oder Nanopigmenten vom Metalloxiden ausgewählt sind, die behandelt wurden oder unbehandelt sind.

12. Zusammensetzung nach Anspruch 11, **dadurch gekennzeichnet, dass** die Pigmente oder Nanopigmente unter den Oxiden von Titan, Zink, Eisen, Zirconium, Cer und deren Gemischen ausgewählt sind, die behandelt wurden oder unbehandelt sind.

13. Zusammensetzung nach einem der Ansprüche 6 bis 12, **dadurch gekennzeichnet, dass** sie ferner ein Bräunungsmittel und/oder Mittel zur künstlichen Braunfärbung der Haut enthält.

14. Zusammensetzung nach einem der Ansprüche 6 bis 13, **dadurch gekennzeichnet, dass** sie ferner mindestens einen Zusatzstoff enthält, der unter den Fettsubstanzen, organischen Lösungsmitteln, ionischen oder nichtionischen, hydrophilen oder lipophilen Verdickungsmitteln, beruhigenden Stoffen, Feuchthaltemitteln, Trübungsmitteln, Stabilisatoren, Emollientien, Siliconen, Schaumverhütungsmitteln, Parfums, Konservierungsmitteln, anionischen, kationischen, nichtionischen, zwitterionischen oder amphoteren grenzflächenaktiven Stoffen, Wirkstoffen, Füllstoffen, Polymeren, Treibmitteln, Alkalisierungsmitteln oder Ansäuerungsmitteln ausgewählt ist.

15. Verwendung einer Zusammensetzung, wie sie in einem der Ansprüche 6 bis 14 definiert ist, für die Herstellung von Produkten zur kosmetischen Behandlung der Haut, der Lippen, der Nägel, der Haare, der Wimpern, der Augenbrauen und/oder der Kopfhaut.

16. Verwendung einer Zusammensetzung, wie sie in einem der Ansprüche 6 bis 14 definiert ist, für die Herstellung von Produkten für die Pflege der Haut, der Lippen, der Nägel, der Haare und/oder der Kopfhaut.

17. Verwendung einer Zusammensetzung, wie sie in einem der Ansprüche 6 bis 14 definiert ist, für die Herstellung von Schminkprodukten.

18. Verwendung eines Silanmerocyaninsulfons, Siloxanmerocyaninsulfons oder Polysiloxanmerocyaninsulfons, wie es in einem der Ansprüche 1 bis 6 definiert ist, in einer kosmetischen oder dermatologischen Zusammensetzung als Stoff, der die UV-Strahlung filtert.

19. Verwendung eines Silanmerocyaninsulfons, Siloxanmerocyaninsulfons oder Polysiloxanmerocyaninsulfons, wie es in einem der Ansprüche 1 bis 6 definiert ist, als Stoff für die Kontrolle der durch die UV-Strahlung verursachten Farbänderung der Haut.

20. Verwendung eines Silanmerocyaninsulfons, Siloxanmerocyaninsulfons oder Polysiloxanmerocyaninsulfons, wie es in einem der Ansprüche 1 bis 6 definiert ist, als Photostabilisator für synthetische Polymere, wie beispielsweise Kunststoffe oder Gläser und insbesondere Brillengläser oder Kontaktlinsen.
